# EUROPÄISCHE PATENTSCHRIFT

(11) **EP 4 601 726 B1**
(45) Veröffentlichungstag und Bekanntmachung des Hinweises auf die Patenterteilung: **25.11.2026**
(21) Anmeldenummer: 23790280.4
(22) Anmeldetag: 13.10.2023
(51) Int. Cl.: A61M 11/04, A61B 5/08, A61K 51/00, A61M 15/02, A61B 5/00, A61B 5/091, A61K 51/12

(54) **VORRICHTUNG UND VERFAHREN ZUR ERZEUGUNG EINES RADIOAKTIV-MARKIERTEN TROCKENAEROSOLS**
DEVICE AND METHOD FOR GENERATING A RADIOLABELED DRY AEROSOL
DISPOSITIF ET PROCÉDÉ DE GÉNÉRATION D'UN AÉROSOL SEC RADIOMARQUÉ

(30) Priorität: 14.10.2022 DE 102022127030
(43) Veröffentlichungstag der Anmeldung: 20.08.2025
(73) Patentinhaber: Almedis Altmann GmbH, 38229 Salzgitter (DE)
(72) Erfinder: ALTMANN, Björn, 38228 Salzgitter (DE); REPARON, Andreas, 38321 Denkte (DE); PAUL, Reiner, 38226 Salzgitter (DE); ALTMANN, Bernd, 38226 Salzgitter (DE); SCHWARZ, Werner, 83324 Rupolding (DE)
(74) Vertreter: Hertin und Partner Rechts- und Patentanwälte PartG mbB
(86) Internationale Anmeldenummer: PCT/EP2023/078528
(87) Internationale Veröffentlichungsnummer: WO 2024/079330

(56) Entgegenhaltungen:
- EP-B1- 0 181 092
- EP-B1- 1 745 803
- CA-A1- 2 245 079
- DE-A1- 102009 015 792

## Beschreibung

Die Erfindung bezieht sich auf eine Vorrichtung und ein Verfahren zur Erzeugung eines radioaktiv-markierten Trockenaerosols. Die Erfindung gehört vorzugsweise zu dem technischen Gebiet der Vorrichtungen und Verfahren zur Durchführung von medizinisch-relevanten Messungen, insbesondere für diagnostische Verfahren im Zusammenhang mit der Untersuchung der Lungenfunktion und der Diagnose von Lungenembolien.

### HINTERGRUND UND STAND DER TECHNIK

Die Verwendung von radioaktiv-markierten Materialien, um biologische und nichtbiologische Prozesse zu analysieren ist aus dem Stand der Technik bekannt. Insbesondere für die Untersuchung der Belüftung der Lunge kann eine Lungenszintigraphie zum Einsatz kommen. Diese Untersuchung wird oft mit einer Untersuchung der Durchblutung der Lunge kombiniert. Insbesondere für die Untersuchung der Belüftung wird ein radioaktiv markiertes Aerosol, beispielsweise aus Technetium-Partikeln, in die Lunge inhaliert. Die Ausbreitung des inhalierten Aerosols in der Lunge wird mit Hilfe einer Gammakamera verfolgt. Sollte diese Untersuchung Lungenbereiche zeigen, die schlecht durchblutet, aber belüftet sind, kann eine Lungenembolie diagnostiziert werden.

Um das radioaktiv markierte Aerosol zu erzeugen, wird eine Vorrichtung zur Aerosolerzeugung verwendet. Ein existierendes Beispiel für eine solche Vorrichtung ist der sogenannte Technegas-Generator. Ein Technetium-Eluat wird in einen Kohlenstofftiegel gefüllt, der erhitzt wird, um das Aerosol zu erzeugen. Der Erhitzungsprozess ist jedoch schwer zu kontrollieren, so dass die produzierten Mengen an Aerosol oft nicht reproduzierbar sind. Außerdem hat sich herausgestellt, dass die Energie, die benötigt wird, um die Kohlenstofftiegel auf eine ausreichend hohe Temperatur zu erhitzen, Stromunterbrechungen in den Stromnetzen verursachen kann. Die verfügbare Stromversorgung beeinflusst auch stark den Betrieb der bekannten Vorrichtung zur Erzeugung des Aerosols.

Die Herstellung des Trockenaerosols hat die hohe Anforderung, dass sie eine Spitzenleistung von weit über 3000 Watt für Sekunden benötigt. Um eine Brennung (weißglühende Erhitzung) des Kohlenstofftiegels bei hoher Brenntemperatur in kürzester Aufheizzeit zu realisieren, wird in der ersten Sekunde der Erhitzung eine Spitzenleistung von weit über 3000 Watt benötigt. Dies setzt eine stabile Stromversorgung beim Anwender voraus, die nicht überall gegeben ist. Ein bekanntes Problem beim Technegas-Generator ist, dass die Sicherung im Strom-Verteilerkasten der Arztpraxis aufgrund der kurzfristig benötigten, sehr hohen Energiemenge auslösen und abschalten kann. Eine solche Abschaltung des Stroms kann weitere Vorrichtungen oder Geräte in der Praxis beeinflussen und ist deshalb gefährlich.

Es ist zudem wichtig, dass die Dosis der radioaktiven Partikel, die dem Patienten während der Untersuchung der Belüftung zugeführt wird, ausreichend, aber begrenzt ist. Dies liegt daran, dass eine zu niedrige Konzentration die Qualität der anschließend erzeugten Gammakamera-Bilder verringert, während eine zu hohe Konzentration die bei der Lungenemboliediagnostik notwendige Durchführung einer anschließenden Untersuchung der Durchblutung mit einem weiteren radioaktiv markierten Material ausschließen kann. Ein Grund dafür ist, dass die Gesamtmenge an Radioaktivität, welche dem Patienten verabreicht werden darf, begrenzt ist.

Bei den bekannten Vorrichtungen sind die Geschwindigkeit, mit der der Kohlenstofftiegel erhitzt wird, und die Geschwindigkeit der Freisetzung des Trockenaerosols von Vorrichtung zu Vorrichtung und von Anwendung zu Anwendung sehr unterschiedlich. Infolgedessen variiert auch die Qualität und Konzentration des Trockenaerosols auf unvorhersehbare Weise. Derzeit gibt es keine bekannte Vorrichtung oder Methode zur Erzeugung eines radioaktiv markierten Aerosols mit gleichbleibender Qualität. Es besteht insbesondere die Notwendigkeit, die Ausbeuteschwankung, d.h. den Unterschied in der Effizienz des Aerosolproduktionsprozesses, zwischen Vorrichtungen und zwischen den Verwendungen der gleichen Vorrichtung zu verringern.

Eine Methode, die in Betracht gezogen wurde, ist die Verringerung der Dicke eines Schutzglases, das die geöffnete Brennkammer zum Anwender abschirmt. Dies ermöglicht eine bessere Sichtbarkeit beim Einfüllen des Eluats des radioaktiven Materials, sodass dies mit höher Präzision in den Kohlenstofftiegel eingefüllt werden kann. Dies verringert jedoch den Schutz des Benutzers, bei dem es sich um einen Arzt, eine Krankenschwester oder einen Techniker handeln kann, der wiederholt radioaktives Material in die Vorrichtung einführt. Es besteht daher Bedarf an einer Lösung, die eine präzise Einbringung und Positionierung der richtigen Menge an Eluat gewährleistet und gleichzeitig ein hohes Maß an Sicherheit für den Anwender bietet.

Außerdem ist die Geschwindigkeit, mit der das radioaktiv markierte Aerosol hergestellt werden kann, und die Konzentration der radioaktiv markierten Partikel derzeit stark begrenzt. Die bekannten Vorrichtungen benötigen bis zu 2 Sekunden, um den Kohlenstofftiegel auf die erforderliche Temperatur für die Freisetzung eines Trockenaerosols zu bringen. Diese Aufheizzeit kann jedoch in der Zeitlänge stark variieren, wodurch sich ein Streuungsmaß ergibt, welches eine Reproduzierbarkeit in der Herstellung des Trockenaerosols erschwert. Ein weiteres Ziel ist daher auch die Entstehung eines solchen Streuungsmaßes stark zu verringern. Die Aufheizzeit ist stark ausschlaggebend für die Qualität und die Partikelmenge des Trockenaerosols. Durch die lange Aufheizzeit erhöht sich die Anzahl der Atemzüge, die ein Patient nehmen muss, um die Lungenszintigraphie durchführen zu können. Es besteht daher auch die Notwendigkeit, das Aerosol mit gleichbleibend hoher Geschwindigkeit und Konzentration zu erzeugen.

Darüber hinaus bieten die derzeit bekannten Vorrichtungen keine Technik, um sicherzustellen, dass die Untersuchung der Belüftung eines Patienten mittels eines radioaktiven Markers sicher und angemessen ist. Dies bleibt dem Fachwissen des Anwenders überlassen. In der Realität haben die Anwender jedoch unterschiedliche Erfahrungswerte und sind möglicherweise nicht in der Lage, die Eignung eines diagnostischen Verfahrens für einen bestimmten Patienten zu beurteilen. Es besteht daher Bedarf an einer Lösung, die eine objektive Beurteilung der Eignung eines Patienten für das Einatmen des radioaktiv markierten Aerosols ermöglicht, bevor dieses verabreicht wird. Das Dokument EP 1 745 803 B1 offenbart relevanten Stand der Technik in Bezug auf die vorliegende Erfindung.

Die bestehende Technologie zur Herstellung eines radioaktiv markierten Aerosols, insbesondere für diagnostische Zwecke, ist daher stark verbesserungsbedürftig.

### AUFGABE DER ERFINDUNG

Es war eine Aufgabe der Erfindung, eine Vorrichtung und ein Verfahren bereitzustellen, welche eine schnelle, sichere und wiederholbare Erzeugung eines radioaktiv-markierten Trockenaerosols gewährleistet. Weiterhin war es eine Aufgabe der Erfindung, eine Vorrichtung und ein Verfahren bereitzustellen, welche eine schnelle, sichere und wiederholbare Bildgebung des alveolaren Raumes ermöglichen. Weitere Aufgaben der Erfindung umfassen die Bereitstellung eines Systems umfassend verschiedene Einwegkomponenten neben einer Vorrichtung für die schnelle, sichere und wiederholbare Erzeugung eines radioaktiv-markierten Trockenaerosols.

### BESCHREIBUNG DER ERFINDUNG UND BEVORZUGTEN

### AUSFÜHRUNGSFORMEN

Gelöst wird die Aufgabe durch die Merkmale der unabhängigen Ansprüche. Vorteilhafte Ausgestaltungen der Erfindung sind in den abhängigen Ansprüchen und in der Beschreibung offenbart.

In einem ersten Aspekt betrifft die Erfindung eine Vorrichtung zur Erzeugung eines radioaktiv-markierten Trockenaerosols umfassend
- einen Kohlenstofftiegel mit einem offenen Hohlraum geeignet zur Befüllung mit radioaktivem Nuklid,
- eine Brennkammer, welche geeignet ist, einen Kohlenstofftiegel aufzunehmen,
- einen Impulsstrom-Geber, welcher eine blitzartige Erhitzung des Kohlenstofftiegels und eines darin enthaltenen radioaktiven Nuklids ermöglicht, sodass ein Trockenaerosol aus radioaktiv-markierten Kohlenstoffpartikeln gebildet wird, welches anschließend zur medizinischen Bildgebung aus der Vorrichtung in die Lunge eines Patienten geleitet werden kann, und
- eine Steuerungseinheit.

Das Füllvolumen des Kohlenstofftiegels beträgt 0,1 mL - 1 mL, vorzugsweise mindestens 0,2 mL, mindestens 0,4 mL, mindestens 0,5 mL oder mindestens 0,6 mL. Vorzugsweise beträgt das Füllvolumen des Kohlenstofftiegels 0,3 - 0,6 mL und besonders bevorzugt 0,4 - 0,5 mL. Weiterhin umfasst die Vorrichtung:
- eine Sonde zur Messung des Sauerstoffgehaltes in der Brennkammer,
- eine Vakuumpumpe zur Entlüftung der Brennkammer,
- einen Druckluftkompressor zur Belüftung der Brennkammer,
- eine Kamera, welche eine Überwachung der Befüllung des Kohlenstofftiegels ermöglicht,
- eine Gamma-Zähler-Messeinheit, welche geeignet ist, eine Veränderung der Radioaktivitätsmenge im Kohlenstofftiegel und/oder der Brennkammer zu messen,
- ein Flow-Meter, welches konfiguriert ist, das Atemvolumen eines Patienten zu bestimmen,
- mindestens eine Batterie und/oder PowerCap/Pufferkondensator und/oder Hochleistungskondensator,
- einen Wechselrichter zur Transformation des Gleichstroms einer Batterie und/oder eines PowerCap/Pufferkondensator und/oder eines Hochleistungskondensators in eine Wechselspannungs-Sinuswelle,
- eine intelligente Netzelektronik-Einheit für einen Stromnetzbetrieb der Vorrichtung mit Trafo, welche konfiguriert ist, die Sinuswelle der Wechselspannung des Netzstroms zu analysieren, wobei die Sinuswelle vorzugsweise eine Standardfrequenz von 50 - 60 Hz aufweist und die Wechselspannung des Netzstroms vorzugsweise 100 - 400 Volt, insbesondere 110 bis 260 Volt, noch bevorzugter 230 Volt beträgt,
- einen externen mit der Vorrichtung verbundener Radioaktivitäts-Sensor, welcher konfiguriert ist, die Radioaktivitätszunahme in der Lunge des Patienten bei einer Verabreichung des erzeugten radioaktiven Trockenaerosols zu erfassen,
- optional eine Vorrichtung zur Integration einer Inert- oder Schutzgas-Quelle, und
- optional einen externen Beatmungsbeutel, welcher an die Vorrichtung angeschlossen werden kann und konfiguriert ist, das Volumen des von einem Patienten eingeatmeten radioaktiv-markierten Trockenaerosols zu vergrößern.

Im Sinne der Erfindung ist eine "Brennung" des Kohlenstofftiegels vorzugsweise eine weißglühende Erhitzung seines Materials. Bei einer solchen Brennung werden Kohlenstoffpartikel aus der Oberfläche des Tiegels vorzugsweise abgespalten. Vorzugsweise wird der Kohlenstoff bei diesem Vorgang teilweise verdampft und sublimiert. Das radioaktive Material (beispielsweise getrocknetes Technetium) an der Oberfläche der Tiegelwanne wird vorzugsweise ebenfalls sublimiert.

Im Sinne der Erfindung ist ein "Trockenaerosol" vorzugsweise eine Suspension aus feinen festen Partikeln in Luft oder einem anderen Gas, wobei die feinen festen Partikel vorzugsweise einen Durchmesser von weniger als 1 µm haben. Zur Veranschaulichung kann Rauch als Beispiel für ein trockenes Aerosol betrachtet werden. Das durch die Erfindung erzeugte Trockenaerosol umfasst vorzugsweise feine Partikel eines radioaktiven Nuklids wie Technetium oder Gallium und Kohlenstoffpartikel in Luft, einem Inertgas oder einer Mischung davon.

Im Sinne der Erfindung ist ein "Nuklid" vorzugsweise ein Atomkern, der durch eine bestimmte Anzahl von Protonen und Neutronen gekennzeichnet ist.

Im Sinne der Erfindung ist ein "PowerCap" vorzugsweise ein "Power Capacitor" oder Leistungskondensator. Dies ist vorzugsweise eine elektrische Vorrichtung, die elektrische Energie speichern und entladen kann. Im Prinzip umfasst dies vorzugsweise ein oder mehrere Paare von Platten, die durch ein isolierendes Material getrennt sind, wobei die Platten an zwei Anschlüssen befestigt sind, die es ermöglichen, die gespeicherte Energie bei Bedarf in einen Stromkreis zu entladen. Ein PowerCap glättet vorzugsweise den Stromfluss durch die Vorrichtung und reduziert so Schwankungen, die eine Schmelzsicherung auslösen oder elektrische Komponenten beschädigen könnten.

Im Sinne der Erfindung ist ein "Pulsstrom" vorzugsweise ein elektrischer Strom, der Pulssequenzen aufweist. Vorzugsweise besteht der Pulsstrom aus kurzen Impulsen, die durch eine längere Pause ohne Stromfluss getrennt sind. Ein solcher Strom kann zum Beispiel von einem Wechselrichter ausgesendet werden. Dem Fachmann sind andere Mechanismen bekannt, die als "Pulsstromgeber" im Sinne der Erfindung dienen können.

In einer Ausführungsform der Vorrichtung ist das Inert- oder Schutzgas ausgewählt aus Argon, Helium, Acetylen und dessen Gasgemische, Stickstoff und Kohlenstoffdioxid. In einer bevorzugten Ausführungsform der Vorrichtung ist das Inert- oder Schutzgas Argon.

In einer bevorzugten Ausführungsform der Vorrichtung ist das radioaktive Nuklid ausgewählt aus Technetium (Tc-99m), Gallium 68, Molybdän-99, Chrom-51, Holmium-166, Rubidium-82, Eisen-59, Lutetium-177, Palladium-103, Kalium-42, Scandium-47, Selen-75, Natrium-24, Xenon-133, Ytterbium-169, Ytterbium-177, Jod-131, Jod-125, Samarium-153, Rhenium-186, Lutetium-177, Phosphor-32 oder Cäsium-131, wobei Technetium (Tc-99m) und Gallium-68 besonders bevorzugt sind.

In einer Ausführungsform wird das Nuklid als Eluat in den Kohlenstofftiegel eingefüllt.

Die Auswahl eines geeigneten Nuklids hängt von verschiedenen Faktoren ab. Mit Gallium 68 ist es möglich, eine Lungenventilationsuntersuchung mit der Bildgebung einer PET-Kamera durchzuführen. PET-Kameras haben eine höhere Auflösung als Gammakameras, welche mit Technetium verwendet werden können. Technetium hat hingegen eine Energie von 133 keV, Gallium von 511 keV. Somit ist Gallium deutlich strahlenintensiver und benötigt daher größere Abschirmungen zum Schutz des Anwenders.

In einer weiteren bevorzugten Ausführungsform der Vorrichtung umfasst der Kohlenstofftiegel Grafit. Vorzugsweise hat Grafit einen sehr hohen Reinheitsgrad, der mindestens 97 wt. %, mindestens 98 wt. %, mindestens 99 wt. %, mindestens 99,7 wt. % oder ganz besonders bevorzugt mindestens 99,9 wt. % beträgt. Der Kohlenstofftiegel kann zum Beispiel ein "Almedis HighVolume-Tiegel" sein. Vorzugsweise hat der Kohlenstofftiegel ein Gewicht von 0,8 - 1,6 g, besonders bevorzugt 1,15 - 1,25 g, insbesondere ca. 1,2 g. Ein bevorzugtes Volumen des Kohlenstofftiegels beträgt mindestens 0,1 mL, vorzugsweise mindestens 0,2 mL, bevorzugter mindestens 0,3 mL, besonders bevorzugt mindestens 0,4 mL. Ein bevorzugtes Volumen des Kohlenstofftiegels beträgt höchstens 1 mL, vorzugsweise höchstens 0,8 mL, besonders bevorzugt höchstens 0,6 mL.

In einer bevorzugten Ausführungsform der Erfindung umfasst die Vorrichtung elektrische Kontakte, welche mit dem Kohlenstofftiegel in Eingriff kommen, um ihn zu erhitzen. Die elektrischen Kontakte umfassen vorzugsweise ein Material, das stromleitfähig ist und das sowohl hohen Temperaturen als auch hohen Strömen standhalten kann. Vorzugsweise sind die elektrischen Kontakte Kohlenstoffkontakte. In einer weiteren Ausführungsform sind die Kontakte in einem Metallblock eingebettet, wobei das Metall aus Messing, Kupfer oder Aluminium ausgewählt sein kann. Die Kontakte können Wechselkontakte sein, welche nach einer vorgegebenen Anzahl an Verwendungen gewechselt werden müssen. Beispielsweise werden die Wechselkontakte nach jeweils 50 Brennungen (50 Erhitzungen des Tiegels) mit einem Schraubenschlüssel aus der Brennkammer entfernt und ersetzt.

In einer weiteren bevorzugten Ausführungsform der Vorrichtung umfasst das Gerät keine Wechselkontakte, die vom Anwender getauscht werden müssen. Die Kontakte können stattdessen Langzeitkontakte, vorzugsweise aus Kohlenstoff sein. Solche Kontakte müssen beispielsweise erst nach jeweils 500 Brennungen gewechselt werden. Dieser Wechsel kann bei einer Wartung der gesamten Vorrichtung durch einen Techniker erfolgen. Eine regelmäßige Ersetzung der Kontakte in kürzen Zeitperioden ist nicht mehr nötig.

In einer weiteren bevorzugten Ausführungsform der Vorrichtung umfasst das radioaktiv-markierte Trockenaerosol Nanopartikel, wobei die Nanopartikel vorzugsweise einen durchschnittlichen Durchmesser zwischen 5 - 250 nm, vorzugsweise 10 - 100 nm, bevorzugter 30 - 60 nm, besonders bevorzugt zwischen 40 - 50 nm, aufweisen. Vorzugsweise weisen mindestens 60%, vorzugsweise mindestens 75%, insbesondere ca. 80% der Nanopartikel einen Durchmesser kleiner als 100 nm auf.

In einer weiteren bevorzugten Ausführungsform umfasst die Vorrichtung Mittel, um den Kohlenstofftiegel automatisch in Kontakt mit elektrischen Kontakten zu bringen, wobei die Mittel vorzugsweise auch für ein automatisches "Einschleifen" zwischen Tiegel und Kontakten konfiguriert sind. Die elektrischen Kontakte sind vorzugsweise so konfiguriert, dass Strom durch den Kohlenstofftiegel fließt, der zwischen ihnen platziert werden kann, so dass der Kohlenstofftiegel als Heizelement fungiert.

Vorzugsweise umfassen die elektrischen Kontakte ein Material das sowohl hohen Temperaturen, insbesondere bis zu 3000 °C, als auch hohen Strömen standhalten kann. Besonders bevorzugt sind die elektrischen Kontakte aus Kohlenstoff. Die elektrischen Kontakte liegen vorzugsweise in einem Aufnahmeblock eingebettet vor. Vorzugsweise umfasst der Aufnahmeblock ein Material, das ebenfalls hohen Temperaturen und hohen Strömen standhalten kann. Besonders bevorzugt umfasst der Aufnahmeblock ein Metall oder eine metallische Legierung, vorzugsweise Messing, Aluminium oder Kupfer.

Die Vorrichtung umfasst vorzugsweise zwei elektrische Kontakte in zwei Aufnahmeblöcken. Die Aufnahmeblöcke sind vorzugsweise mit elektrischen Kabeln eines Stromkreises verbunden.

Die Vorrichtung dient vorzugsweise der Erzeugung eines Trockenaerosols, insbesondere aus ultrafeinen Kohlenstoffpartikeln. Die Kohlenstoffpartikeln sind vorzugsweise mit dem radioaktiven Nuklid "markiert". Vorzugsweise umfassen die Partikel des Trockenaerosols sechseckige Plättchen aus Kohlenstoff. Vorzugsweise lagert sich das radioaktive Nuklid an die sechseckigen Plättchen, um diese zu markieren. Vorzugsweise beträgt ein durchschnittliches Verhältnis der Schichtdicke zum Durchmesser der sechseckigen Plättchen 1: 20 - 1:5, insbesondere ca. 1:10. Dieses Trockenaerosol eignet sich vorteilhafterweise zur Einatmung, insbesondere um die Lungenfunktion eines Patienten mit einer Gamma-Kamera und/oder PET-Kamera zu erfassen. Hierdurch kann beispielsweise eine Lungenembolie, möglicherweise der Schweregrad einer akuten oder chronisch obstruktiven Atemwegserkrankung quantifiziert werden und/oder möglicherweise ebenso der Grad der Obstruktionen in Covid 19 Patienten diagnostiziert werden. Weiterhin kann das Trockenaerosol bei postoperativen Untersuchungen, insbesondere bei Lungenvolumenreduktionen zum Einsatz kommen. In einer bevorzugten Ausführungsform kann das Trockenaerosol bei präoperativen und postoperativen Untersuchungen zum Einsatz kommen.

Mittels der Vorrichtung kann der Kohlenstofftiegel in weniger als 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden auf 2500 °C - 3000 °C, insbesondere ca. 2750 °C aufgeheizt werden. Dies erfolgt vorzugsweise in einer sauerstofffreien Atmosphäre. Diese Zeitdauer wird im Sinne der Erfindung vorzugsweise als "Risetime" bezeichnet. Nach der Risetime wird die Temperatur (auch "Brenntemperatur" im Sinne der Erfindung bezeichnet) des Kohlenstofftiegels auf 2500 °C - 3000 °C, insbesondere ca. 2750 °C, 10 - 20 Sekunden lang gehalten. Vorzugsweise wird der Kohlenstofftiegel bei ca. 2750 °C für 13 - 17 Sekunden, insbesondere ca. 15 Sekunden lang gehalten. Während der Risetime und des Zeitraums der aufrechterhaltenen Erhitzung wird vorzugsweise das Trockenaerosol erzeugt. Die Energie zum Erhöhen und Aufrechterhalten der Temperatur des Kohlenstofftiegels wird vorzugsweise durch Netzstrom oder durch Batteriebetrieb bereitgestellt.

Es kann bevorzugt sein, dass der Kohlenstofftiegel bei seiner Erhitzung mit Netzstrom gespeist wird. In diesem Fall kann es vorteilhaft sein, einen Triac und/oder Transformator (Trafo) einzusetzen, um den elektrischen Strom und/oder die Spannung zu regulieren, die dem Kohlenstofftiegel zugeführt werden. Es kann auch bevorzugt werden, dass die Erhitzung des Kohlenstofftiegels batteriebetrieben ist. In diesem Fall kann es vorteilhaft sein, eine Pulsweitenmodulation mit Nulldurchgang einzusetzen, um die Stromzufuhr zum Kohlenstofftiegel zu regulieren.

Es ist besonders bevorzugt, dass ein Energiespeicher für die Aufheizung verwendet wird. Als Beispiel können Batterien, PowerCaps und/oder Hochleistungskondensatoren zum Einsatz kommen. Hierdurch kann der kurzfristige Energiebedarf ausreichend gedeckt werden, ohne eine Instabilität in einem Netzwerk zu erzeugen. Potenziell gefährliche Stromausfälle können vermieden werden. Eingebaute Batterien können insbesondere eine stabile Energieversorgung zum Kohlenstofftiegel beim Brennvorgang ermöglichen, insbesondere auch um eine dauerhafte Risetime-Verkürzung auf weniger als 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden zu ermöglichen.

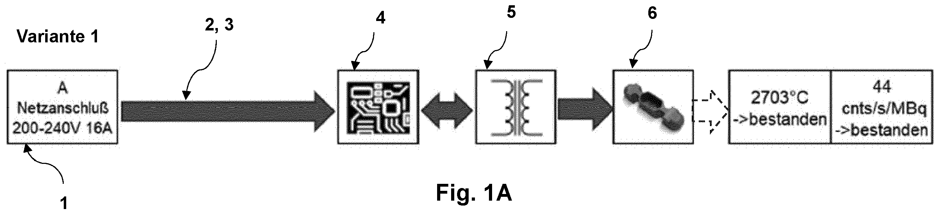

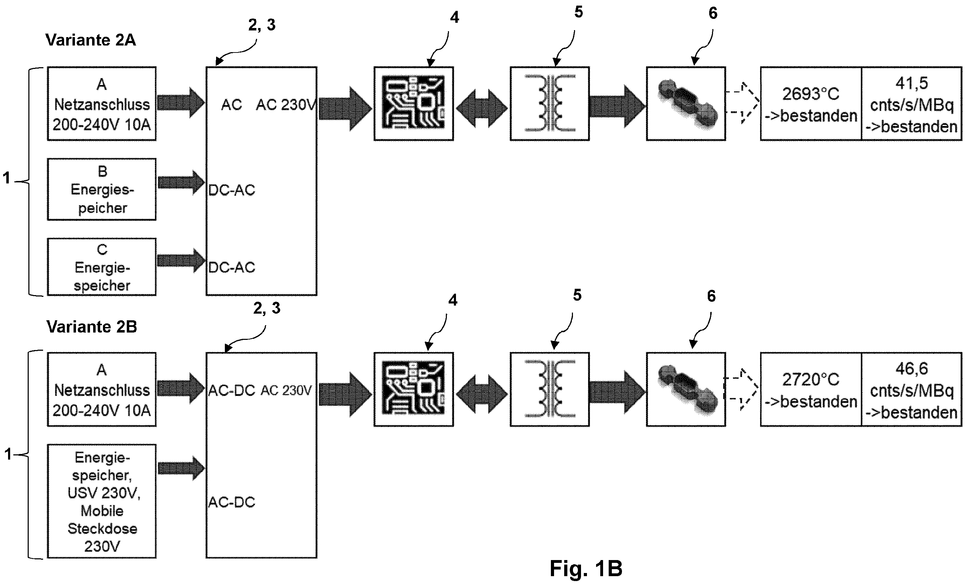

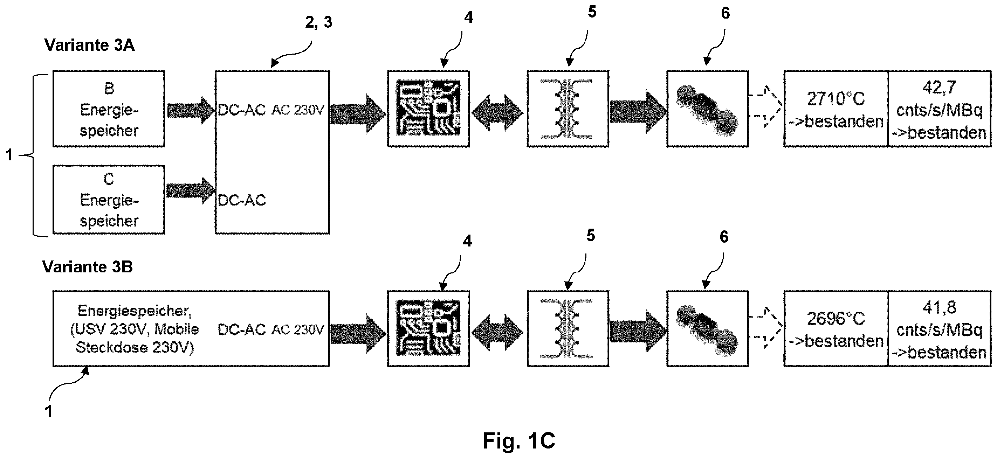

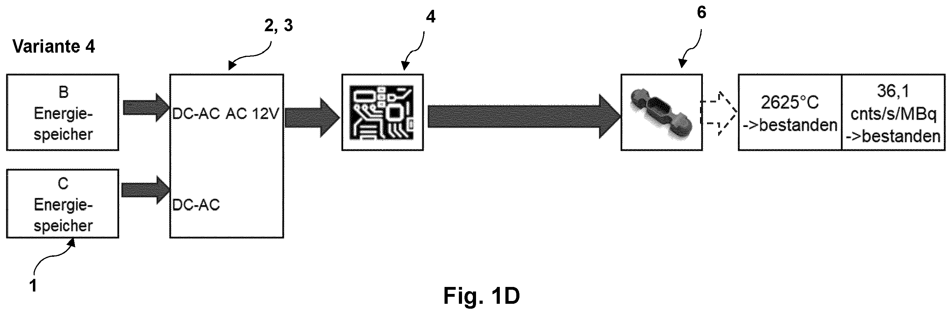

Für die Energieversorgung des Kohlenstofftiegels sind verschiedene Varianten möglich. Bei einer ersten Variante A wird der Netzstrom nur zum Laden von Batterien oder PowerCaps benötigt. Diese Variante A entspricht Variante 3A, 3B und 4 in Fig. 1. Das Aufladen stellt keine große Herausforderung für die vorhandene Strominstallation des Anwenders dar. Durch den Batterie- oder PowerCaps-Betrieb kann dann das Trockenaerosol stromnetzunabhängig in jedem Raum hergestellt werden. Die Gleichspannung der Batterie oder des PowerCaps wird vorzugsweise in eine ca. 10 Volt Hochstrom-Wechselspannung transformiert.

Mittels Pulsweitenmodulation / Wechselrichter wird aus den Gleichstromquellen eine Sinuswelle / Wechselspannung mit der benötigten hohen Energiemenge (ca. 600 Ampere) generiert, welche die erforderliche schnelle Risetime des Tiegels ermöglicht.

Bei einer zweiten Variante B (entspricht Variante 2A und 2B in Fig. 1 wird der Netzstrom während der Risetime durch die Batterien oder PowerCaps unterstützt / geboostert. Diese Variante B entspricht Variante 2A und 2B in Fig. 1. Diese Kombination aus Netzstrom und Energiespeicher stellt somit keine große Herausforderung an die vorhandene Strominstallation des Benutzers dar. Durch den Batterie- oder PowerCaps-Energiebooster kann somit das Trockenaerosol mit fast jedem Stromanschluss hergestellt werden. Die Gleichspannung der Batterie oder des PowerCaps / Energiespeichers wird in eine 230 Volt Wechselspannung mittels Pulsweitenmodulation / Wechselrichter umgewandelt. Die aus den Gleichstromquellen / Energiespeicher gewandelte Wechselspannung wird auf die Sinuswelle des Netzeingangs im Gerät (Steckdosen-Strom) nahezu in Echtzeit aufgeschaltet und boostert / steigert die 230 Volt Netzstromstärke auf weit über 16 Ampere. Hierdurch steht einem Trafo die kurzfristig benötigte hohe Energie für die erforderliche schnelle Risetime des Tiegels beim Fullburn sicher zur Verfügung.

In einer bevorzugten Ausführungsform der Erfindung analysiert eine intelligente Powerstrom-Elektronik die Sinuswelle eines 100 bis 400 Volt, vorzugsweise 110 bis 260 Volt-, insbesondere 230 Volt-Netzstrom und schaltet den Power-Trafo zur Aufheizung des Kohlenstofftiegels erst ein, wenn die Einschaltlast des Trafos einen definierten, günstigen Abschnitt auf der 50 Hz Sinuswelle des Netzstroms erreicht. Vorzugsweise ist ein günstiger Abschnitt der Sinuswelle ein vorbestimmter Bereich von Phasenlagen, in dem ein Einschaltrush beim Einschalten des Transformators vermieden werden kann. Der durchschnittliche Fachmann ist in der Lage, einen solchen günstigen Abschnitt zu identifizieren und Steuermittel zum Einschalten des Transformators zum richtigen Zeitpunkt bereitzustellen. Hierdurch wird gewährleistet, dass dem Trafo die benötigte sehr hohe Startenergiemenge für das Aufheizen des Tiegels (Risetime) in kürzester Zeit aus einer Standard-230V-Steckdose zur Verfügung steht, ohne dass die elektrische Sicherung der Strominstallation des Anwenders auslöst.

In bevorzugten Ausführungsformen der Erfindung werden elektrische Kontakte für die Aufheizung des Kohlenstofftiegels auf die Brenntemperatur mit einem elektrischen Strom von bis zu 600A bei einer Spannung von 7 - 15, vorzugsweise etwa 10 Volt gespeist. Vorzugsweise wird dieser Strom für die Dauer der Risetime angewendet und dann auf einen Strom von 200 - 400A, vorzugsweise 300A reduziert, während die Temperatur des Kohlenstofftiegels auf der Brenntemperatur gehalten wird. Vorzugsweise wird die Risetime mithilfe eines Erfassungsgeräts und/oder der Steuereinheit optimiert, sodass diese kurz gehalten wird. Dies kann durch eine intelligente Steuerung eines Powerboards bei einer Energieversorgung aus einer Standardsteckdose erfolgen.

In weiteren bevorzugten Ausführungsformen der Erfindung ist die Stromfrequenz variierbar. Vorzugsweise kann die Stromfrequenz zwischen 50 - 4000 Hz eingestellt werden. Hierdurch kann die Effizienz der Vorrichtung verbessert werden.

Ein optimiertes Aufheizmanagement des Kohlenstofftiegels kann auch dazu beitragen, die Streuung der Trockenaerosolqualität zu reduzieren. Hierdurch wird eine Ausbeuteschwankung des Kohlenstofftiegels geringgehalten.

In bevorzugten Ausführungsformen der Erfindung wird die Brenntemperatur weniger als 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden erreicht. Dies erhöht die Ausbeute (Menge der Radioaktivität in Partikelform im Trockenaerosol) des Kohlenstofftiegels. Hierdurch kann weniger Eluat und/oder weniger Atemzüge des Patienten für die Erfassung einer Lungenfunktion notwendig sein.

In einer weiteren bevorzugten Ausführungsform der Erfindung umfasst die Vorrichtung ein Erfassungsgerät für die Überwachung der Trockenaerosolerzeugung in der Brennkammer. Vorzugsweise erfolgt eine Kammeraktivitätsmessung zur Überwachung ("Monitoring") der entstandenen Radioaktivitätsmenge in der Brennkammer. Diese Information kann an die Steuereinheit weitergeleitet werden und/oder zur Unterstützung des Benutzers optional verarbeitet und auf ein Display gezeigt werden. Vorzugsweise umfasst das Erfassungsgerät eine Gamma-Counter-Messeinheit. Hierdurch wird gewährleistet, dass eine ausreichende Menge an radioaktiven Partikeln im erzeugten Trockenaerosol vorhanden ist. Das Risiko eines Anwenderfehlers, beispielsweise einer zu frühen Verabreichung des Trockenaerosols oder eine unzureichende Befüllung des Kohlenstofftiegels, wird ebenfalls minimiert. Ein solcher Fehler kann zum Misslingen einer medizinischen Untersuchung führen.

In bevorzugten Ausführungsformen der Erfindung umfasst die Vorrichtung Mittel zur Temperaturmessung des Kohlenstofftiegels, insbesondere während und nach seiner Aufheizung. Es kann besonders bevorzugt sein, dass die Temperaturmessung durch Messung der Lichtintensität des glühenden Kohlenstofftiegels erfolgt. Dies ist besonders vorteilhaft für eine präzise Messung ohne Kontakt zum Tiegel bzw. der des erzeugten Aerosols, so dass das erzeugte Aerosol weder verunreinigt noch der Sensor beschädigt wird. Alternativ kann eine Pyrometer-Messeinheit verwendet werden.

In einer weiteren bevorzugten Ausführungsform der Erfindung liegt die Temperatur des erzeugten radioaktiv-markierten Trockenaerosols oder des Inertgases zwischen 10 - 40 °C, vorzugsweise zwischen 15 - 30 °C. Bei dieser Temperatur kann das Gas sicher und bequem vom Patienten inhaliert werden, ohne Verbrennungen zu verursachen und ist gleichzeitig sicher für die Verwendung mit den verschiedenen Teilen der Vorrichtung, z.B. Einwegschläuchen und Ventilen. Es wurde auch festgestellt, dass bei diesen Temperaturen überraschend wenig Verschleiß an den festen Teilen der Vorrichtung wie der Brennkammer auftrat.

Vorzugsweise ist die Vorrichtung nach außen abgeschirmt, um die Umgebung und alle darin befindlichen Anwender und/oder Patienten vor radioaktiver Strahlung zu schützen. Die Abschirmung kann vorzugsweise mittels Bleis erfolgen und insbesondere die Brennkammer umgeben. In weiteren Ausführungsform kann die Abschirmung vorzugsweise mittels Bleis und/oder Wolfram erfolgen und insbesondere die Brennkammer umgeben. In einigen Ausführungsformen erfolgt die Abschirmung mittels eines Bleigehäuses.

In bevorzugten Ausführungsformen der Erfindung umfasst die Vorrichtung Mittel zum manuellen Eingriff in die Steuereinheit, vorzugsweise ein Touchscreen. Vorzugsweise umfasst die Vorrichtung auch eine Ausgabeeinheit wie z.B. ein Display, welches die durchzuführende Ablaufschritte eines Verfahrens anzeigt. Hierdurch kann die Vorrichtung besonders anwenderfreundlich konfiguriert sein.

In einer bevorzugten Ausführungsform der Erfindung wird das Eluat, welches in dem Kohlenstofftiegel gefüllt wird getrocknet. Vorzugsweise bedeutet dies, dass jegliches Wasser aus dem Eluat entfernt wird. Vorzugsweise wird Wasserdampf, Raumluft und/oder Sauerstoff durch einen Evakuierungsprozess entfernt, z.B. durch das Abpumpen von Wasserdampf, Sauerstoff und/oder Luft aus der Brennkammer.

In bevorzugten Ausführungsformen der Erfindung wird eine Kombination aus Kohlenstoff, vorzugsweise aus dem Kohlenstofftiegel, und einem inerten Gas, vorzugsweise Argon, verwendet, um getrocknetes Technetium Eluat (Natriumpertechnetat) in einer Kammer zu verdampfen oder atomisieren.

Vorzugsweise wird die Brennkammer vor dem Erhitzen des Kohlenstofftiegels mit einem Inertgas gefüllt. Dies kann geschehen, indem Inertgas in die Kammer gepumpt wird, um das Gas / die Luft in der Kammer zu verdrängen, das dann durch einen Ausgangskanal entweichen kann. Dieser Schritt ist vorzugsweise so konfiguriert, dass jeglicher Sauerstoff aus der Brennkammer entfernt wird. Geeignete Inertgase sind Argon oder Stickstoff, wobei Argon besonders bevorzugt wird.

Es kann bevorzugt sein, dass die Vorrichtung Mittel zur Zuführung des Inertgases von einem Druckbehälter zu der Brennkammer umfasst. Solche Mittel können einen Kanal, ein steuerbares Ventil und/oder eine steuerbare Pumpe umfassen. Vorzugsweise liegt der Druckbehälter auf einem bewegbaren Trolley, welcher Teil der Vorrichtung ist oder mit der Vorrichtung verwendet werden kann.

Vorzugsweise umfasst die Vorrichtung Mittel zur Erzeugung von Druckluft und/oder eines Vakuums. Dies kann zum Beispiel eine Pumpe und/oder einen Druckbehälter umfassen.

Vorzugsweise umfasst die Vorrichtung eine Batterie oder eine Akkustromquelle, die in einem optionalen Schritt der Verabreichung des Trockenaerosols an einen Patienten eingesetzt werden kann. Dies ist besonders vorteilhaft, um sicherzustellen, dass die Vorrichtung in dieser Phase frei beweglich ist.

In einer weiteren bevorzugten Ausführungsform umfasst die Vorrichtung ein Mundstück, einen Patientenschlauch, ein Patienten-Auslassventil zur selektiven Abgabe von Gas oder Trockenaerosol aus der Brennkammer in den Patientenschlauch und/oder ein Belüftungsventil zum selektiven Einlass von Raumluft in die Brennkammer, um einen Strom durch die Brennkammer in den Patientenschlauch zu erzeugen. Ein "Patientenschlauch" im Sinne der Erfindung ist vorzugsweise ein Schlauch, in dem das Aerosol von der Brennkammer für die Anwendung am Patienten geleitet wird. Vorzugsweise sind das Mundstück und der Patientenschlauch als Einwegkomponente konfiguriert.

In einer weiteren bevorzugten Ausführungsform umfasst die Vorrichtung ein Auslassventil zur Evakuierung der Brennkammer, insbesondere zur Entfernung von Wasserdampf, Restsauerstoff und Spülgas aus der Brennkammer. In einer weiteren bevorzugten Ausführungsform umfasst die Vorrichtung ein Einlassventil zur Befüllung der Brennkammer mit einem inerten Gas und/oder mit Druckluft.

In einer bevorzugten Ausführungsform der Vorrichtung ist die Brennkammer mit einer verschließbaren Öffnung zum manuellen Einführen von Werkzeugen, z.B. einer Spritze, ausgestattet, insbesondere um den Kohlenstofftiegel zu füllen.

In einer weiteren bevorzugten Ausführungsform der Erfindung umfasst die Vorrichtung Mittel für den Fernzugriff eines Technikers, insbesondere auf die Steuereinheit und etwaige Eingabe- oder Rückmeldeeinheiten wie Touchscreen, Display, Tastatur, Alarm, Licht, etc. Vorzugsweise enthält die Vorrichtung auch Mittel zur Verbindung mit einem lokalen oder drahtlosen Netzwerk, insbesondere eine Bluetooth-, Funk-, Satelliten- oder WLAN-Verbindung.

In einer weiteren bevorzugten Ausführungsform der Erfindung umfasst die Vorrichtung einen Datenspeicher. Vorzugsweise ist die Steuereinheit dafür konfiguriert, Daten von einem oder mehreren Erfassungsgeräten im Datenspeicher zu speichern. Hierdurch kann der Verlauf eines Verfahrens über die Vorrichtung automatisch protokolliert werden. Werte wie z.B.: eingefüllten Aktivitätsmenge, Temperaturerreichung und Verlauf, Anzahl der Atemzüge, Volumen der Atemzüge, gemessene Aktivität in der Kammer und der Aktivitätszunahme in der Lunge können pro Patient von der Vorrichtung erfasst und protokolliert werden. Diese Werte sollen als Protokoll der Untersuchung dienen können.

In einem zweiten Aspekt betrifft die Erfindung ein Verfahren zur Erzeugung von radioaktiv-markiertem Trockenaerosol umfassend
a. Bereitstellen einer Vorrichtung zur Erzeugung von radioaktiv-markiertem Trockenaerosol gemäß dem ersten Aspekt der Erfindung,
b. Durchführung eines Atemtests (Pre-Breathing) des Pateienten, wobei das Atemvolumen des Patienten zur Beurteilung seiner Untersuchungsfähigkeit gemessen wird,
c. Kamera-überwachtes Befüllen eines in die Brennkammer der Vorrichtung eingesetzten Kohlenstofftiegels mit einem radioaktiven Nuklid,
d. optionaler Drucktest zur Kontrolle der Dichtigkeit der Brennkammer,
e. optionales Verdampfen einer Trägerflüssigkeit des radioaktiven Nuklids,
f. optionales Trocknen des radioaktiven Nuklids,
g. Evakuieren der Luft aus der Brennkammer zur Erzeugung eines Vakuums,
h. Befüllen der Brennkammer mit Argongas, mit gleichzeitiger Überwachung von eventuell vorhandenem Restsauerstoff, zur Erzeugung einer inerten Argon-, Stickstoff- oder Schutzgas-Atmosphäre in der Brennkammer,
i. Vorwärmen des Kohlenstofftiegels auf vorzugsweise 500°C,
j. Erhitzung des befüllten Kohlenstofftiegels auf 2.500 - 3.000 °C, vorzugsweise 2.750 °C zur Erzeugung eines radioaktiv-markierten Trockenaerosols oder Nanopartikel-Komposits, wobei der während des Aufheizens benötigte Strom zur Verfügung gestellt wird, entweder
   I. durch die enthaltene mindestens eine Batterie und/oder PowerCap/Pufferkondensator und/oder Hochleistungskondensator, oder
   II. durch Netzstrom, welcher durch die mindestens eine Batterie und/oder PowerCap/Pufferkondensator und/oder Hochleistungskondensator unterstützt und optional verstärkt wird, oder
   III. durch Netzstrom, wobei die intelligente Netzelektronik-Einheit die Sinuswelle (vorzugsweise 50Hz) der Wechselspannung des Netzstroms (vorzugsweise 230 Volt) analysiert, und den Trafo erst einschaltet, wenn die Einschaltlast des Trafos einen vordefinierten, günstigen Zeitpunkt auf der Sinuswelle des Netzstroms erreicht,
   sodass eine Erhitzung des befüllten Kohlenstofftiegels auf 2.500 - 3.000 °C, vorzugsweise 2.750 °C vorzugsweise binnen bis zu 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden erreicht wird,
k. optionale Überwachung der Veränderung der Radioaktivität in der Brennkammer,
l. Lungen-Aktivitäts-Überwachung während der Inhalation des erzeugten radioaktiv-markierten Trockenaerosols oder Nanopartikel-Komposits durch einen Patienten, wobei die vom Patienten inhalierte Radioaktivitätsmenge durch einen, vorzugsweise am Patienten lokalisierten, Radioaktivitäts-Sensor überwacht wird,
m. Spülung der Brennkammer mit Druckluft zur Entfernung der radioaktiven Partikel, wobei die radioaktiven Partikel von einem Filter aus dem ausströmenden Luft-, Argon-, Stickstoff- oder Schutzgasgemisch entfernt werden, und optionale Überwachung der Veränderung der Radioaktivität in der Brennkammer,
n. optionale automatische Protokollierung einer oder mehrerer Vorgangsparameter.

In einer bevorzugten Ausführungsform des Verfahrens wird für jeden Durchgang des Verfahrens ein neuer Kohlenstofftiegel in die Brennkammer eingesetzt. Das Einsetzen des Kohlenstofftiegels in die Brennkammer erfolgt vorzugsweise manuell. Es kann auch von Vorteil sein, dass die Vorrichtung Mittel zur automatischen Positionierung des Kohlenstofftiegels in der Brennkammer umfasst und der Kohlenstofftiegel automatisch mit elektrischen Kontakten in Eingriff gebracht wird.

In einer bevorzugten Ausführungsform des Verfahrens ist das radioaktive Nuklid ausgewählt aus Technetium (Tc-99m) oder Gallium 68.

In einer weiteren bevorzugten Ausführungsform des Verfahrens liegt das radioaktive Nuklid in Form eines flüssigen Eluats vor. Vorzugsweise wird das Eluat manuell in den Hohlraum des Kohlenstofftiegels eingebracht. Vorzugsweise wird dieser Schritt von einer Kamera überwacht. Vorzugsweise wird ein live überwachtes Bild der Kamera auf einem Display angezeigt. Dies ermöglicht nicht nur eine bessere Sicht und die Möglichkeit, das überwachte Bild für eine bessere Präzision zu vergrößern, sondern erhöht auch die Sicherheit des Benutzers, da keine Körperteile, sondern nur Werkzeuge in die Brennkammer gelangen müssen. Da die Brennkammer in einer dicken Bleiabschirmung eingeschlossen sein kann, wird die Sicht durch die Kamera erheblich verbessert, so dass Verschüttungen, Abfälle und Schäden an der Vorrichtung vermieden werden.

Vorzugsweise kann der Benutzer auf ein Display schauen, welche ihm/ihr das Livebild seiner Tätigkeit in der Geräteschublade per Kamera in Echtzeit auf dem Monitorscreen anzeigt. Per variablem Zoom kann er/sie bei Bedarf seine Handhabungen visuell vergrößern. Durch die Kamera-Überwachung kann der sonst notwendige Blick durch das dicke abschirmende Bleiglas entfallen. Die nötige Strahlenschutz-Abschirmung zum Anwender kann nun problemlos durch eine entsprechende Bleiplattenabschirmung realisiert werden, wodurch auch Nuklide höherer Energie wie z.B. Gallium zum Einsatz kommen können.

In einer weiteren bevorzugten Ausführungsform des Verfahrens wird ein Drucktest zur Kontrolle der Dichtigkeit der Brennkammer durchgeführt. Vorzugsweise wird die Brennkammer mit Luft oder einem inerten Gas bei einem Überdruck von 75 - 500 mbar, vorzugsweise 100 - 300 mbar, insbesondere ca. 120 mbar gefüllt. Die Brennkammer umfasst vorzugsweise verschließbare Ventile, welche nach der Füllung mit Luft oder einem inerten Gas geschlossen werden. Der Verlauf des Druckabfalls wird beobachtet, z.B. mittels eines Druckmessers. Die Daten des Druckmessers werden vorzugsweise an die Steuereinheit geliefert. Sollte die Geschwindigkeit des Druckabfalls einen vorgegebenen Wert überschreiten, löst die Steuereinheit vorzugsweise eine Fehlermeldung aus. Dies kann in Form eines Alarms, eines Lichtsignals, einer Meldung auf einem Display oder ähnlichem geschehen.

In einer weiteren bevorzugten Ausführungsform des Verfahrens wird während einer Verdampfungsphase das Wasser aus dem Kohlenstofftiegel verdampft. Das Wasser kann den größten Teil des Eluats ausmachen und hat ungefähr das gleiche Volumen wie das Eluat. Wenn beispielsweise 0,3 ml Eluat in einen Kohlenstofftiegel mit einem Fassungsvermögen von 0,3 ml gegeben werden, ist das Volumen des verdampften Wassers vorzugsweise so nahe an 0,3 ml, dass es als 0,3 ml angenähert werden kann. Vorzugsweise wird der Kohlenstofftiegel während der Verdampfungsphase auf 70 - 95 °C, vorzugsweise ca. 85 °C, erhitzt. Vorzugsweise dauert diese Phase 3 - 6 Minuten, besonders bevorzugt ca. 4 Minuten. Während dieser Zeit wird der entstehende "Wasserdunst" durch die strömende Druckluft aus der Kammer befördert und entweicht der Brennkammer durch ein geöffnetes Auslassventil, welches vorzugsweise hinter einem Gerätefilter in der Stromrichtung angeordnet ist.

In einer weiteren bevorzugten Ausführungsform des Verfahrens wird während einer Sicherheitstrocknungsphase (auch "TTM" oder Tiegel-Temperatur-Management im Sinne der Erfindung) ein eindiffundierter Restwasseranteil durch Erwärmen des Kohlenstofftiegels auf 150 - 200 °C, insbesondere ca. 180 °C aus jeglichen Poren des Kohlestofftiegels ausgetrieben. Vorzugsweise dauert dieser Vorgang 0,5 - 2 Minuten, insbesondere ca. 1 Minute. Während dieser Phase wird der entstehende "Wasserdunst" (Wasserdampf) durch durchströmende Druckluft aus der Brennkammer gefördert und entweicht durch das geöffnete Auslassventil hinter dem Gerätefilter.

In einer weiteren bevorzugten Ausführungsform umfasst die Vorrichtung eine Vakuumpumpe und/oder einen Kompressor zur Ressourcenschonung von dem inerten Gas. Vorzugsweise wird bei dem Verfahren die Luft aus der Brennkammer abgepumpt. Hierdurch kann ein Vakuum in der Brennkammer entstehen. Der anschließende Aufbau einer Schutz-Atmosphäre in der Brennkammer wird damit verkürzt und erleichtert. Die benötigte Menge an Argon oder Inertgas kann so stark reduziert und somit Verbrauchsmittelkosten gesenkt werden.

Vorzugsweise wird ein Kompressor in der Brennkammer integriert. Der Kompressor kann vorzugsweise Druckluft erzeugen. Abläufe in der Brennkammer, für die kein inertes Schutzgas nötig ist, laufen nun mit einfacher gefilterter Druckluft ab. Die Druckluft erzeugt die Vorrichtung selbst. Die benötigte Menge an Inertgas, insbesondere Argon, kann stark reduziert und somit Verbrauchsmittelkosten gesenkt werden.

In einer weiteren bevorzugten Ausführungsform des Verfahrens wird die Brennkammer mit einem inerten Gas, vorzugsweise mit Argon gefüllt. Nach diesem Schritt bleibt vorzugsweise kein Sauerstoff in der Brennkammer. Vorzugsweise wird der Brennkammer ein Überschuss an Inertgas zugeführt, wobei ein Teil des Inertgases die Brennkammer über das offene Auslassventil verlässt. Durch dieses Verfahren kann die sauerstoffhaltige Luft in der Brennkammer durch das Inertgas verdrängt werden, so dass kein Sauerstoff mehr übrigbleibt.

In einer weiteren bevorzugten Ausführungsform des Verfahrens wird ein Sauerstoffanteil in der Brennkammer und/oder in einem Auslasskanal aus der Brennkammer überwacht. Vorzugsweise werden gemessene Daten des Sauerstoffanteils an die Steuereinheit geliefert. In einem Beispiel wird bei einem Kammerausgang eine Sauerstoffmesssonde eingesetzt. Vorzugsweise gibt die Steuereinheit ein Signal aus, wenn der ermittelte Sauerstoffanteil einen vorgegebenen Grenzwert über- oder unterschreitet. Beispielsweise wird eine Meldung auf ein Display gezeigt. Nur wenn der Sauerstoffanteil unter den vorgegebenen Grenzwert fällt, kann der Kohlenstofftiegel auf die Brenntemperatur erhitzt werden.

In einer weiteren bevorzugten Ausführungsform des Verfahrens werden jegliche Ventile der Brennkammer vor der Erhitzung des Kohlenstofftiegels auf die Brenntemperatur geschlossen.

In einer weiteren bevorzugten Ausführungsform umfasst das Verfahren den Schritt des Vorwärmens des Kohlenstofftiegel. Vorzugsweise wird der Kohlenstofftiegel zunächst auf eine Temperatur zwischen 250 - 800 °C, bevorzugt 350 - 650 °C, besonders bevorzugt ca. 500 °C, erhitzt. Dies reduziert die Risetime und verbessert die Effizienz des Verfahrens.

In einer weiteren bevorzugten Ausführungsform des Verfahrens wird der Kohlenstofftiegel durch schlagartiges Erhitzen auf die Brenntemperatur geheizt. Vorzugsweise beträgt die Brenntemperatur 2500 - 3000 °C, insbesondere ca. 2750 °C. Vorzugsweise wird die Brenntemperatur innerhalb einer Risetime von bis zu 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden erreicht.

In einer weiteren bevorzugten Ausführungsform des Verfahrens wird die Risetime reduziert. Vorzugsweise wird die Risetime von bis zu 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden mithilfe einer intelligenten Powerstrom-Elektronik mit einem Netz- und/oder Energiespeicher betrieb umgesetzt. Vorzugsweise kann das Erhitzen mit Hilfe eines Akkumulators, einer Batterie, einer PowerCap, eines Pufferkondensators und/oder eines Hochleistungskondensators durchgeführt werden.

In einer weiteren bevorzugten Ausführungsform des Verfahrens während der Erhitzung des befüllten Kohlenstofftiegels unter Punkt j. der Gleichstrom der mindestens einen Batterie und/oder PowerCap/Pufferkondensator und/oder Hochleistungskondensators in eine Wechselspannung (mit vorzugsweise 230 Volt) mittels Pulsweitenmodulation oder Wechselrichter umgewandelt wird, sodass die aus der mindestens einen Batterie und/oder PowerCap/Pufferkondensator und/oder Hochleistungskondensator gewandelte Spannung auf die Sinuswelle der Wechselspannung (vorzugsweise 230 Volt) des Stromnetzeingangs der Vorrichtung aufgeschaltet wird und somit die Netzstromstärke auf vorzugsweise ≥ 16 Amper verstärkt.

In einer weiteren bevorzugten Ausführungsform des Verfahrens wird eine entstandene Radioaktivitätsveränderung in der Brennkammer durch ein oder mehr Erfassungsgeräte überwacht. Vorzugsweise werden die erfassten Daten an die Steuereinheit weitergeleitet. Vorzugsweise gibt die Steuereinheit Signale aus, um das Erreichen einer bestimmten Phase im Prozess der Herstellung des Trockenaerosols anzuzeigen. Es kann auch bevorzugt sein, dass die Steuereinheit ein Display anweist, dem Benutzer auf der Grundlage der erfassten Daten eine Nachricht anzuzeigen. Sobald festgestellt wird, dass radioaktiv markiertes Trockenaerosol im Wesentlichen erzeugt wird (z.B. wenn der Grad der Radioaktivität einen vorbestimmten Schwellenwert überschreitet), wird das Trockenaerosol vorzugsweise innerhalb von 15 Minuten, noch bevorzugter innerhalb von 10 Minuten, an eine Lunge verabreicht.

In einer weiteren bevorzugten Ausführungsform des Verfahrens während des Atemtests unter Punkt b. der Patient durch ein, vorzugsweise an die Vorrichtung angeschlossenes, Schlauchset atmet, sodass beim Einatmen gefilterte Raumluft durch ein, vorzugsweise in der Vorrichtung integriertes, Flow-Meter strömt, welches das eingeatmete Volumen pro Atemzug misst.

In einer weiteren bevorzugten Ausführungsform des Verfahrens wird die Brennkammer belüftet, sodass ein Patient das radioaktiv-markierte Trockenaerosol in ein bis fünf Atemzügen einatmen kann. Die Belüftung erfolgt vorzugsweise durch ein Belüftungsventil, welches selektiv Luft in die Brennkammer einführen lassen. Das radioaktiv-markierte Trockenaerosol wird vorzugsweise durch ein geöffnetes Patienten-Auslassventil und einen "Patientenschlauch" aus der Brennkammer zu dem Patienten geführt. Bei jedem Atemzug strömt durch das geöffnete Belüftungsventil Raumluft in die Kammer nach. Ist ausreichend viel Aktivität in der Lunge, wird das Patienten-Auslassventil geschlossen und die Verabreichung ist beendet.

In einer weiteren bevorzugten Ausführungsform des Verfahrens wird das tatsächliche Atemvolumen des Patienten zur Beurteilung seiner Untersuchungsfähigkeit gemessen. Die Daten von dieser "Pre-Breathing"-Messung können an die Steuereinheit geführt und auf dem Display gezeigt werden.

Es ist ein weltweit bekanntes Problem, dass der Anwender im Umgang mit einem radioaktiv-markierten Trockenaerosol oft vorher nicht genau weiß, ob sein Patient überhaupt in der Lage ist, die nötigen 1 bis 5 tiefen Atemzüge mit ausreichendem Einatmungsvolumen auszuführen. Erfahrene Anwender können dies besser abschätzen als unerfahrene. Es kommt häufiger vor, dass die Prozedur abgebrochen werden muss. Hier setzt unser neues Pre-Breathing-Monitoring an. Als Vorbereitung atmet der Patient durch das angeschlossene Schlauchset. Hierbei strömt beim Einatmen gefilterte Raumluft (ohne Aktivität) durch einen Bypass an der Brennkammer vorbei in den Patientenschlauch. Ein integriertes Flow-Meter misst das eingeatmete Volumen pro Atemzug und unterstützt den Anwender bei der Entscheidung, ob das Atemvolumen des Patienten für eine erfolgreiche Untersuchung ausreicht. Dieses Vorgehen spart dem Anwender Zeit und ist für den Patienten sicherer. Der Patient läuft nicht Gefahr sich bezüglich einer abgebrochenen ergebnislosen Untersuchung mit Radioaktivität belastet zu haben. Für die Anwender wird insbesondere die Zeit für Fehluntersuchungen gespart.

Sollte der Patient auch nach gezielter Aufforderung nicht ausreichend tief atmen können, kann der Anwender gleich einen unterstützenden Beatmungsbeutel am Gerät einsetzen.

Im Kontext der vorliegenden Erfindung ist der Beatmungsbeutel vorzugsweise ein Beutel, der zusammengedrückt werden kann, um Luft durch die Brennkammer und den Patientenschlauch zu drücken und dem Patienten zuzuführen. Dies ist besonders nützlich, wenn der Patient nicht in der Lage ist, genügend Saugkraft aufzubringen, um diesen Luftstrom zu erzeugen.

Wenn der Beatmungsbeutel zusammengedrückt wird, öffnet sich vorzugsweise automatisch ein Ausgangsventil im Beutel. Die Luft wird aus dem Beutel in die Brennkammer gedrückt. Vorzugsweise öffnet der Luftstrom automatisch ein Ventil, das den Durchfluss durch den Patientenschlauch ermöglicht. Das radioaktiv markierte Aerosol wird so in ausreichender Menge an den Patienten abgegeben, bevor die Ausatmung erfolgen kann.

Zur Ausatmung des Patienten wird der Beatmungsbeutel vorzugsweise freigegeben. Der Beatmungsbeutel umfasst vorzugsweise Mittel, um sich selbst wieder aufzublasen, z.B. ein spezielles Aufblasventil. Dieser Vorgang kann so oft wiederholt werden, wie es für sicher und notwendig gehalten wird.

In einer weiteren bevorzugten Ausführungsform des Verfahrens wird die inhalierte Menge an Trockenaerosol in einer Lunge überwacht. Dies kann mithilfe eines Detektionsgeräts oder Sensors zur Erfassung des Zerfalls von Positronen pro Zeit erfolgen. Die Ergebnisse können in einem Speicher gespeichert und/oder auf ein Display gezeigt werden.

In einer weiteren bevorzugten Ausführungsform des Verfahrens wird nach einmaliger Verwendung des Kohlenstofftiegels das Trockenaerosol aus der Brennkammer gespült. Vorzugsweise erfolgt die Spülung mit Druckluft oder Inertgas aus einem Druckbehälter oder mithilfe eines Kompressors. Die Druckluft oder das Inertgas können das Trockenaerosol verdrängen, das vorzugsweise sicher entsorgt wird. Dies erfolgt vorzugsweise mithilfe eines bleigeschirmten Gerätefilters. Das Verfahren kann vorzugsweise auch einen Schritt der mechanischen Zerstörung des Kohlenstofftiegels und/oder der radioaktiv geschützten Lagerung und Entsorgung von Einwegkomponenten wie einem Rohr zur Abgabe des Trockenaerosols an einen Patienten umfassen.

In einer weiteren bevorzugten Ausführungsform des Verfahrens ist ein Erfassungsgerät ("Aktivitäts-Sensor") zur Erfassung der radioaktiven Aktivität, insbesondere zur Erfassung des Zerfalls von Positronen pro Zeit, in einer Weste angebracht. Die Weste kann mit der erfindungsgemäßen Vorrichtung verwendet werden. Mithilfe der Weste kann die Aktivitätszunahme in der Lunge des Patienten bei der Verabreichung des radioaktiven Trockenaerosols erfasst werden. Bei der Untersuchung, vorzugsweise um eine Ventilationsszintigraphie durchzuführen, trägt der Patient diese Weste, die vorzugsweise mit der Vorrichtung verbunden ist.

Im Kontext der vorliegenden Erfindung ist die Weste vorzugsweise dadurch, dass sie mehrere räumlich verteilte Sensoren und Mittel zur Befestigung an der Vorderseite, der linken Seite, der rechten Seite und/oder der Rückseite des Thoraxbereichs des Patienten umfasst. Die Weste kann z.B. als Lätzchen mit Nackenband, eine Schürze, als ein vergrößerter Gürtel, als Hemd oder als Kleidungsstück mit einer Öffnung für den Kopf zwischen einem vorderen und einem hinteren Teil ausgeführt sein. Vorzugsweise umfasst die Weste verstellbare Befestigungsmittel, um eine enge Passform für Patienten mit unterschiedlichen Körperformen und -größen zu ermöglichen. Vorzugsweise umfasst die Weste eine Öffnung für den Kopf, die sich zwischen einem Vorder- und einem Rückenteil befindet, sowie Klettverschlüsse, mit denen die Vorder- und Rückenteile an den Seiten und/oder unter den Achseln des Patienten befestigt werden können, um einen guten Sitz zu gewährleisten.

Vorzugsweise wird ein Zielwert der gewünschten Aktivitätsmenge in der Lunge des Patienten von dem Benutzer vorgegeben. Dieser Wert ist individuell und z.B. auch abhängig von der Gamma-Kamera und/oder PET-Kamera, die der Anwender anschließend zur Bildgebung nutzen kann.

Der Benutzer definiert also vorzugsweise bei der Verabreichung das "Zielkriterium", um zu gewährleisten, dass nur die gewünschte Aktivitätsmenge in die Lunge gelangt. Die Ventile und/oder Pumpen der Vorrichtung können von der Steuereinheit auf der Grundlage dieser vorbestimmten Daten gesteuert werden, um sicherzustellen, dass gerade genug Trockenaerosol in der Lunge des Patienten ankommt, um die gewünschte Analyse/Untersuchung durchzuführen, aber nicht mehr als notwendig, um den Patienten nicht unnötig mit Radioaktivität zu belasten. Auch für einen optionalen zweiten Teil der Untersuchung, der Perfusionsszintigraphie zur Darstellung der Durchblutung der Lunge, ist es vorteilhaft, dass nicht zu viel radioaktiv-markiertes Trockenaerosol bei der Ventilationsszintigraphie in die Lunge gelangt ist, da sonst die zulässige Gesamtstrahlendosis überschritten werden könnte und der zweite direkt folgende Untersuchungsteil (Perfusionsszintigraphie) zu diesem Zeitpunkt nicht durchgeführt werden dürfte und zeitlich verschoben werden müsste.

Wird der gewünschte Aktivitäts-Sollwert vom Sensor auf der Brust des Patienten gemessen, gibt die Steuereinheit ein Signal. Das Patientenventil wird geschlossen. Keine weitere Aktivität kann mehr eingeatmet werden. Die Verabreichung des radioaktiven Trockenaerosols wird beendet.

In einem dritten Aspekt betrifft die Erfindung eine Verwendung der erfindungsgemäßen Vorrichtung zur bildgebenden Darstellung der durchlüfteten Bereiche einer Lunge, vorzugsweise mithilfe einer Gammakamera und/oder PET-Kamera.

In einer bevorzugten Ausführungsform der Erfindung erfolgt die bildgebende Darstellung der durchlüfteten Bereiche einer Lunge mittels Lungenventilation-Szintigraphie. Es kann bevorzugt sein, dass die bildgebende Darstellung eine tomographische Darstellung ist.

Die erfindungsgemäße Vorrichtung kann in bestimmten Ausführungsformen auch zum Aufspüren von Verstopfungen und/oder Lecks in einem nicht-lebenden Luftstromsystem, z.B. in einem Mikrostromreaktor, verwendet werden. Auch bei einer solchen Anwendung kann die Erfindung zur bildgebenden Darstellung einer System-Durchlüftung verwendet werden, vorzugsweise mithilfe einer Gammakamera und/oder PET-Kamera.

In einem vierten Aspekt betrifft die Erfindung eine Vorrichtung umfassend eine Brennkammer, einen Impulsstrom-Geber, eine Steuereinheit, eine Sonde zur Messung des Sauerstoffgehaltes in der Brennkammer, eine Vakuumpumpe, einen Druckluftkompressor, eine Kamera und eine Energieversorgungseinheit wie oben beschrieben. Insbesondere in diesem Aspekt ist der Kohlenstofftiegel nicht Teil der Vorrichtung, sondern eine Verbrauchskomponente, die mit der Vorrichtung verwendet werden kann. Vorzugsweise ist die Vorrichtung so konfiguriert, dass sie verschiedene Formen und Größen von Kohlenstofftiegeln aufnehmen kann.

In einem fünften Aspekt betrifft die Erfindung ein System zur Erzeugung eines radioaktiv-markierten Trockenaerosols umfassend, wobei das System eine Vorrichtung gemäß dem vierten Aspekt umfasst in Kombination mit einem Kohlenstofftiegel, einem Druckluftbehälter, einem Inertgas-Druckbehälter, einem Einwegpatientenschlauch und/oder einem externen Beatmungsbeutel.

Der durchschnittliche Fachmann erkennt, dass technische Merkmale, Definitionen und Vorteile bevorzugter Ausführungsformen des erfindungsgemäßen Verfahrens auch für die erfindungsgemäße Vorrichtung und das erfindungsgemäße System gelten, und umgekehrt.

Begriffe wie im Wesentlichen, ungefähr, etwa, ca., usw. beschreiben vorzugsweise einen Toleranzbereich von weniger als ± 20 %, vorzugsweise weniger als ± 10 %, besonders bevorzugt weniger als ± 5 % und insbesondere weniger als ± 1 % und schließen den genauen Wert ein.

### Beispiele

### Beispiel 1:

### Übersicht der Messergebnisse durchgeführter Vorversuche je Versuchsvariante gemäß der Figur 1

### 1. Beschreibung des Versuchsaufbaus und -Ablaufs der durchgeführten Tests zur Erzeugung des Trockenaerosols

Bauteile:
- Test-Brennkammer mit Dom
- Test-Stromquellen
- Test-Power-Elektronik
- Test-Steuerungselektronik
- Kohlenstofftiegel
- Kohlenstoff-Kontakte
- Salzlösung
- Inertgas / Argon
- Sauerstoffmesseinheit
- Druckpumpe
- Vakuumpumpe
- Test-Filter
- Radioaktives Nuklid z.B. Technetium-Eluat

### Versuchsablauf Teil 1

Es wurde mit Salzlösung ohne ein radioaktives Nuklid getestet. Außerdem wurde die jeweils verwendete elektrische Einrichtung aus Energiequelle und Elektronik auf Funktion und Aufheizgeschwindigkeit des Tiegels getestet. Diese Tests wurden im Elektroniklabor durchgeführt und dienen der Überprüfung der elektro-thermischen Funktion der jeweiligen Variante.

### Versuchsablauf Teil 2

Es wurde mit Salzlösung und mit einem radioaktiven Nuklid (Technetium) getestet. getestet. Außerdem wurde die jeweils verwendete elektrische Einrichtung aus Energiequelle und Elektronik auf Funktion und Aufheizgeschwindigkeit des Tiegels getestet, sowie die abgegebene Radioaktivität als radioaktiv markierte Kohlenstoffpartikel. (Die Tests mit Radioaktivität konnten nur in einer Nukleareinrichtung z.B. Nuklearmedizinischer- Abteilung durchgeführt werden.)

| | |
|---|---|
| • Vorbereitung | Einsetzen des Kohlenstoff-Tiegels in die Brennkammer Manuelles Befüllen des Tiegels mit aktiven Eluat (max. 0,3 ml) |
| | *Neu: Kamerabasiertes manuelles Befüllen des Tiegels Innovativer Anwenderschutz* / *Strahlenschutz* |
| • Dichtigkeitstest | Drucktest zur Kontrolle der Dichtigkeit der Kammer. Die Kammer wird mit einem Überdruck von 120 mbar gefüllt. |
| | Alle Ventile werden geschlossen und der Verlauf des |
| | Druckabfalls wird ausgewertet. Bei zu schnellem Druckabfall erfolgt eine Fehlermeldung des Geräts zur Kammerdichtigkeit. |
| • Eindampfen | Der Wasseranteil (ca. 0,3 ml) wird durch Erwärmen des Tiegels auf ca. 85°C aus dem radioaktiven Eluat entfernt. Der Vorgang dauert ca. 4 Minuten. Während dieser Zeit wird der entstehende "Wasserdunst" durch die strömende Druckluft aus der Kammer befördert und entweicht durch das geöffnete Geräte-Auslassventil hinter dem Gerätefilter. |
| *• Trocknen* | *Neu: Sicherheitstrocknung (TTM Tiegel- Temperatur-Management)* |
| | *Der eindiffundierte Restwasseranteil wird durch Erwärmen des Tiegels auf ca.* 180°C *aus den Poren ausgetrieben. Der Vorgang dauert ca. 1 Minute. Während dieser Zeit wird der entstehende "Wasserdunst" durch die strömende Druckluft aus der Kammer befördert und entweicht durch das geöffnete Geräte-Auslassventil hinter dem Gerätefilter.* |
| • *Evakuieren (NeuerProzess-Schritt)* | |
| | *Neu: Integration einer Vakuumpumpe und Kompressor zur Ressourcenschonung von Argongas.* Die Luft wird aus der Kammer abgepumpt. Es entsteht ein Vakuum in der Kammer. |
| • Inert Atmosphäre | Die Kammer wird mit Argon gefüllt, es darf kein Sauerstoff in der Kammer verbleiben. Überschüssiges Argon entweicht durch das geöffnete Geräte-Auslassventil hinter dem Gerätefilter. |
| | Neu: Das aus dem geöffneten Geräte-Auslassventil ausströmende Argon wird mit Hilfe einer SauerstoffMesseinheit auf Restsauerstoff kontrolliert. |
| • Schließen der Venti Ventile | Die Kammer ist mit inerter Schutzgasatmosphäre drucklos gefüllt. |
| *• Vorwärmen (NeuerProzess-Schritt)* | |
| | *Neu: Vorwärmen des Tiegels auf z.B.* 500°C *(zur Beschleunigung der anschließenden Risetime)* |
| • Brennvorgang | Das Ventigas-Trockenaerosol wird durch schlagartiges Erhitzen des Tiegels auf ca. 2750°C erzeugt. Die Aufheizzeit / Risetime soll dabei nur noch 0,5 bis 0,8 Sekunden dauern! |
| | *Neu: Rise-Time-Beschleunigung (Reduzierung der Aufheizzeit des Tiegels)* |
| | *Neu: Intelligente Powerstrom-Elektronik mit Netz und* / *oder Energiespeicher-Betrieb* |
| | *Neu: Kammer-Aktivitäts-Monitoring Zum Monitoren der entstandenen Radioaktivitätsveränderung in der Gerätekammer zur Unterstützung des Anwenders (Assistenzsystem zur Unterstützung des Anwenders)* |
| | Hinweis: Das Zeitfenster für eine Verabreichung an den Patienten ab Brennvorgang ist max. 10 Minuten. Die Konzentration der Partikel im Gasgemisch sinkt über die Zeit! Bei den Versuchen werden die Proben nach einer Minute nach dem Brennvorgang gezogen. |
| • Verabreichung / bei Versuchen Absaugung in den Testfilter | |
| | Absaugen des aktiven Ventigas-Trockenaerosols durch das geöffnete Patienten-Auslassventil und einen "Patientenschlauch" aus der belüfteten Kammer in einen Testfilter. Beim Absaugen strömt durch das geöffnete Belüftungsventil Raumluft in die Kammer nach. Nach dreifachem Kammervolumen-Luftaustausch sind die freien Partikel mit der Aktivität im Testfilter. Das Patienten-Auslassventil wird geschlossen. |
| • Aktivitätsbewertung | *Auswertung der Radioaktivitätsmenge der abgesaugten Partikel im Testfilter im Vergleich zur Standartanwendung.* |
| • Spülen der Kammer | Restliches aktives Ventigas wird aus der Generatorkammer durch Spülung ***mit Druckluft aus dem neuen* Gerätekompressor** entfernt. Restliche schwebende Partikel mit Aktivität werden dabei in den Gerätefilter gespült und sicher ausgefiltert. Argon und Luft entweichen über das geöffnete Geräte-Auslassventil. |
| | *Neu: Integration eines Kompressors zur Ressourcenschonung von Argongas.* |
| | *Neu: Kammer-Aktivitäts-Monitoring Zum Monitoren der entstandenen Radioaktivitätsveränderung in der Gerätekammer.* |

### 2. Überprüfung der verschiedenen Varianten an Stromquellen und Elektronikaufbauten zur Erzeugung der radioaktiv markierten Kohlenstoffpartikel / des radioaktiven Trockenaerosols (Varianten gemäß Figure 1)

Die Tests der verschiedenen Varianten (Figure 1) an Stromquellen und Elektronikaufbauten zur Erzeugung der radioaktiv markierten Kohlenstoffpartikel / des radioaktiven Trockenaerosols wurden in nuklearmedizinischen Einrichtungen durchgeführt. Bei diesen Versuchen werden nur die technischen nuklearmedizinischen Gerätschaften und radioaktive Nuklide genutzt. Patienten kamen mit den Tests nicht in Kontakt.

Nuklearmedizinischen Einrichtungen:
Harzer PET-Zentrum & Nuklearmedizin
Dr. med. Frank Straube
Kösliner Straße 12
38642 Goslar

Evangelisches Krankenhaus Bethesda zu Duisburg
Klinik für Nuklearmedizin
Heerstraße 219
47053 Duisburg

**Tabelle 1: Übersicht der Messergebnisse der durchgeführten Vorversuche je Variante nach Figur 1**

| | Messung 1 | Messung 2 | Messung 3 | **Mittelwerte** | |
|---|---|---|---|---|---|
| Standardanwendung zum Vergleich | 2720 | 2700 | 2660 | **2693,3** | **°C** |
| | 36,9 | 42,7 | 51,1 | **43,6** | **cnts/s/M Bq** |
| Variante 1 | 2730 | 2710 | 2670 | **2703,3** | **°C** |
| | 41,0 | 45,5 | 45,6 | **44,0** | **cnts/s/M Bq** |
| Variante 2A | 2700 | 2690 | 2690 | **2693,3** | **°C** |
| | 40,2 | 46,7 | 37,8 | **41,5** | **cnts/s/M Bq** |
| Variante 2B | 2724 | 2740 | 2696 | **2720,0** | **°C** |
| | 43,8 | 48,9 | 47,0 | **46,6** | **cnts/s/M Bq** |
| Variante 3A | 2750 | 2690 | 2690 | **2710,0** | **°C** |
| | 44,1 | 43,7 | 40,4 | **42,7** | **cnts/s/M Bq** |
| Variante 3B | 2710 | 2700 | 2680 | **2696,7** | **°C** |
| | 42,0 | 44,1 | 39,4 | **41,8** | **cnts/s/M Bq** |
| Variante 4 | 2650 | 2600 | 2627 | **2625,7** | **°C** |
| | 36,1 | 27,2 | 45,1 | **36,1** | **cnts/s/M Bq** |

### Beispiel 2:

### Vergleichsmessungen zur Darstellung der Wirksamkeit der entwickelten "intelligenten Powerstrom-Elektronik (IPE)" im Trockenaerosolgenerator

Versuche mit einem Funktionsmuster haben ergeben, dass beim "unkontrollierten, zufälligen" Einschalten des Transformators beim Brennvorgang der Einschaltstrom ein Vielfaches des Nennstroms betragen kann.

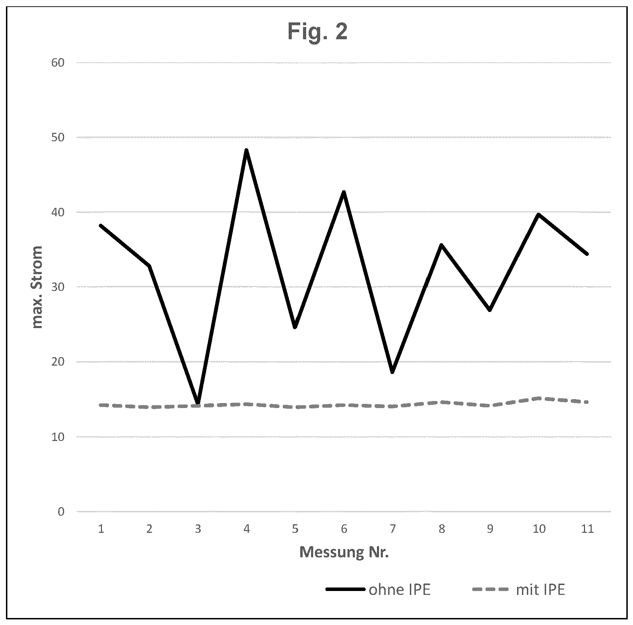

Weitere Versuche haben gezeigt, dass durch das "kontrollierte" Ein- und Ausschalten durch eine intelligente Steuerung der Einschaltstrom soweit reduziert werden konnte, dass dieser nicht mehr wesentlich vom Nennstrom abweicht (siehe Tabelle 2 und Fig. 2).

Die Messreihen zeigen den großen Unterschied des Einschaltstroms in der Höhe und Streuung beim Einschalten des Transformators zum Brennvorgang. Eine Messreihe ist ohne und eine mit der Verwendung der "intelligenten Powerstrom-Elektronik (IPE)" durchgeführt worden.

**Tabelle 2:**

| Messung Nr. | ohne IPE (in Ampere) | mit IPE (in Ampere) |
|---|---|---|
| 1 | 38,2 | 14,2 |
| 2 | 32,8 | 13,9 |
| 3 | 14,3 | 14,1 |
| 4 | 48,3 | 14,3 |
| 5 | 24,6 | 13,9 |
| 6 | 42,7 | 14,2 |
| 7 | 18,6 | 14 |
| 8 | 35,6 | 14,6 |
| 9 | 26,9 | 14,1 |
| 10 | 39,7 | 15,1 |
| 11 | 34,4 | 14,6 |

### Beispiel 3:

### Kammeraktivitätsmessung zur Überwachung ("Monitoring") der entstandenen Radioaktivitätsmenge in der Brennkammer

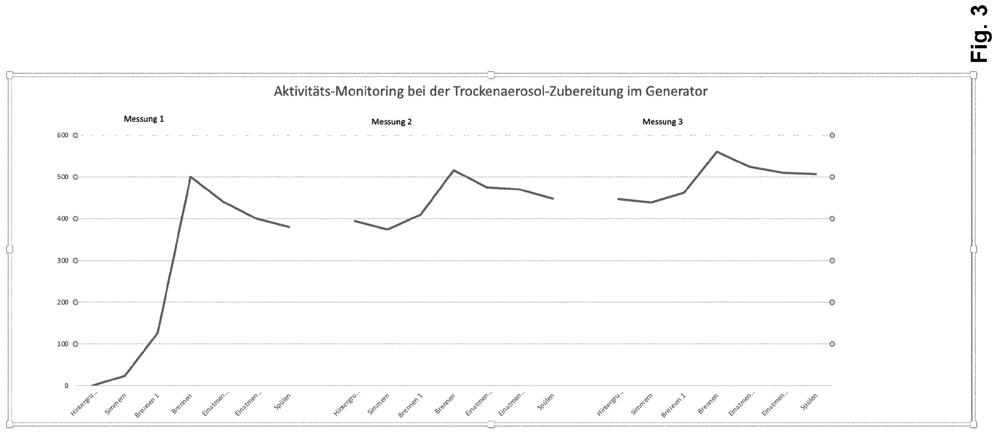

Versuche mit einer Brennkammer, wie sie vorzugsweise in der bevorzugten Ausführung der Erfindung verwendet wird, haben gezeigt, dass sich die Zunahme der Aktivität in der Kammer mittels einer Gamma-Counter-Messeinheit ermitteln und auswerten lässt. Dadurch ist durch Verwendung einer entsprechenden Auswerte Software eine Erfassung der Aktivitätszunahme, und somit eine Beurteilung der Aktivitätsausbeute des erzeugten Trockenaerosols möglich, was auf die Qualität des Trockenaerosols schließen lässt (siehe Fig. 3).

### Beispiel 4

### Überwachung des vorhandenen Restsauerstoffgehaltes in der Brennkammer

Weiterhin wurde eine Verlaufsmessung zur Überwachung des Restsauerstoffsgehaltes beim Aufbau der Inertgas- / Schutzgasatmosphäre in der Brennkammer des Trockenaerosol-Generators / Ventigas-Generators durchgeführt, d.h. beim Befüllvorgang der Brennkammer mit Argongas.

Ziel ist die Erzeugung einer inerten Argon-, Stickstoff- oder Schutzgas-Atmosphäre in der Brennkammer, um den anschließenden Erhitzungsprozess des Kohlenstofftiegels ohne die Anwesenheit von Sauerstoff durchführen zu können. Um sichere Aussagen zur Qualität der Schutzgasatmosphäre tätigen zu können und einen stabilen reproduzierbaren Prozess zu gewährleisten, ist eine stetige Qualitätskontrolle der Schutzgasatmosphäre notwendig.

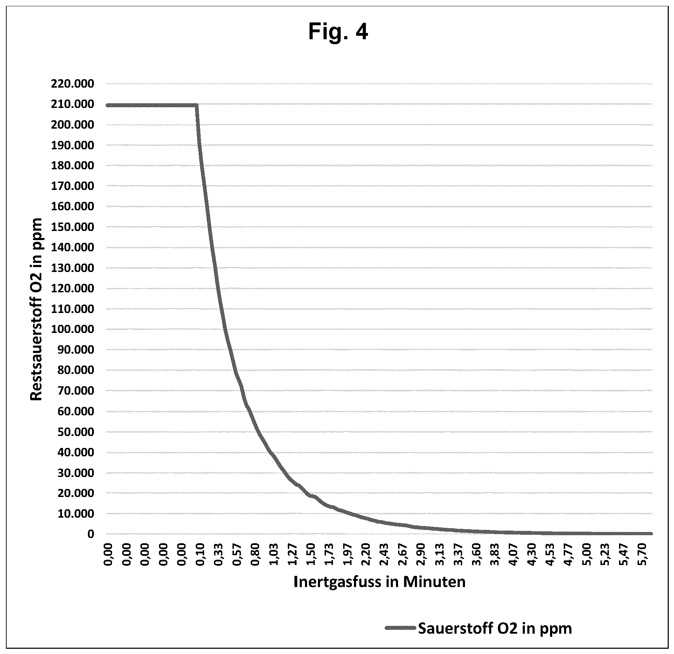

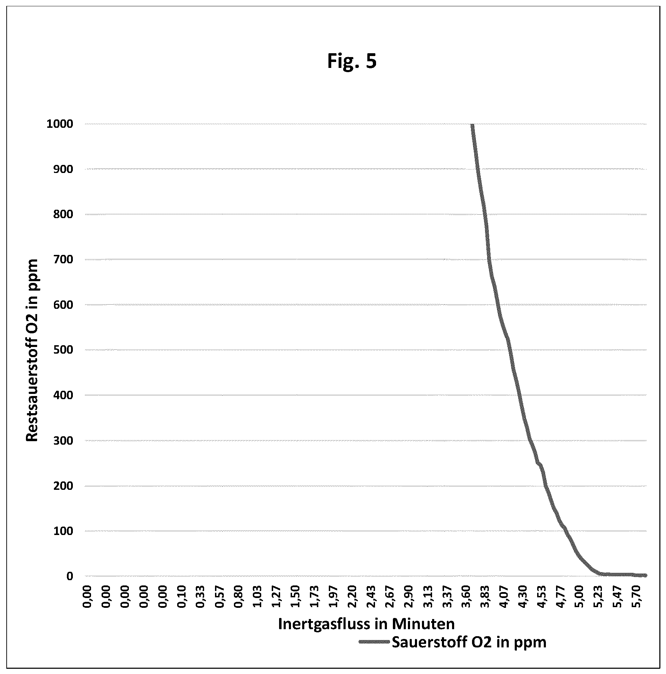

Zum Nachweis der Umsetzbarkeit wurde in einem Versuch in den Testaufbau der Gerätekammer eine geeignete Sauerstoff-Messzelle integriert. Mit dieser Messzelle konnte im Versuch der Sauerstoffgehalt in der Brennkammer beim Aufbau der Schutzgasatmosphäre wie benötigt quantifiziert und bewertet werden (siehe Tabelle 3 und Fig. 4 und 5).

Die Messreihe zeigt den Verlauf des Gehaltes an Restsauerstoff in der Brennkammer beim Aufbau der Schutzgasatmosphäre. Der Restsauerstoffgehalt wird in ppm (parts per million / Teilchen pro Million) angegeben. Über die Zeit nimmt der Restsauerstoffgehalt immer weiter ab und die Konzentration der Schutzgasatmosphäre nimmt zu. Mit der neuen Messeinrichtung lässt sich exakt der Zeitpunkt bestimmen, zu dem ein vorgegebener extrem niedriger Grenzwert erreicht wird und der nächste Prozessschritt starten kann. Dies ist für eine kontinuierliche und reproduzierbare hohe Qualität des Trockenaerosols von großer Bedeutung.

**Figure 4** zeigt die Messreihe als grafische Messkurve in Form eines Diagramms über den gesamten Messverlauf des Restsauerstoffgehaltes beim Aufbau der Inertgas-Schutzgasatmosphäre in der Brennkammer des Trockenaerosol-Generators. Die "Auflösung" des Diagramms in y-Richtung (O2 in ppm) ist hier grob in 10.000-der Schritten angezeigt.

**Figure 5** zeigt die Messreihe als grafische Messkurve in Form eines Diagramms über das Ende des Messverlaufs des Restsauerstoffgehaltes beim Aufbau der Inertgas-Schutzgasatmosphäre in der Brennkammer des Trockenaerosol-Generators. Die "gezoomte Auflösung" des Diagramms in y-Richtung (O2 in ppm) ist hierbei feiner in 100-ter Schritten angezeigt.

**Tabelle 3:**

| Messzeit | Restsauerstoff | | |
|---|---|---|---|
| Zeit in Sek. | Sauerstoff O2 in ppm | Inertgasfluss in Sek. | Inertgasfluss in Min. |
| 0 | 209400 | 0 | 0,00 |
| 2 | 209400 | 0 | 0,00 |
| 4 | 209400 | 0 | 0,00 |
| 6 | 209400 | 0 | 0,00 |
| 8 | 209400 | 0 | 0,00 |
| 10 | 209400 | 0 | 0,00 |
| 12 | 209400 | 0 | 0,00 |
| 14 | 209400 | 0 | 0,00 |
| 16 | 209400 | 0 | 0,00 |
| 18 | 209400 | 0 | 0,00 |
| 20 | 209400 | 0 | 0,00 |
| 22 | 209400 | 0 | 0,00 |
| 24 | 209400 | 0 | 0,00 |
| 26 | 209400 | 0 | 0,00 |
| 28 | 209400 | 0 | 0,00 |
| 30 | 209400 | 0 | 0,00 |
| 32 | 209400 | 0 | 0,00 |
| 34 | 209400 | 0 | 0,00 |
| 36 | 209400 | 0 | 0,00 |
| 38 | 209400 | 0 | 0,00 |
| 40 | 209400 | 0 | 0,00 |
| 42 | 209400 | 0 | 0,00 |
| 44 | 209400 | 0 | 0,00 |
| 46 | 209400 | 0 | 0,00 |
| 48 | 209400 | 0 | 0,00 |
| 50 | 209400 | 0 | 0,00 |
| 52 | 209400 | 0 | 0,00 |
| 54 | 209400 | 0 | 0,00 |
| 56 | 209400 | 0 | 0,00 |
| 58 | 209400 | 0 | 0,00 |
| 60 | 209400 | 0 | 0,00 |
| 62 | 209400 | 0 | 0,00 |
| 64 | 209400 | 0 | 0,00 |
| 66 | 209400 | 2 | 0,03 |
| 68 | 209400 | 4 | 0,07 |
| 70 | 191503 | 6 | 0,10 |
| 72 | 179621 | 8 | 0,13 |
| 74 | 169930 | 10 | 0,17 |
| 76 | 159990 | 12 | 0,20 |
| 78 | 149027 | 14 | 0,23 |
| 80 | 139231 | 16 | 0,27 |
| 82 | 131049 | 18 | 0,30 |
| 84 | 121411 | 20 | 0,33 |
| 86 | 113444 | 22 | 0,37 |
| 88 | 106578 | 24 | 0,40 |
| 90 | 99074 | 26 | 0,43 |
| 92 | 93774 | 28 | 0,47 |
| 94 | 89138 | 30 | 0,50 |
| 96 | 83868 | 32 | 0,53 |
| 98 | 78404 | 34 | 0,57 |
| 100 | 75384 | 36 | 0,60 |
| 102 | 72226 | 38 | 0,63 |
| 104 | 66836 | 40 | 0,67 |
| 106 | 62990 | 42 | 0,70 |
| 108 | 60728 | 44 | 0,73 |
| 110 | 57699 | 46 | 0,77 |
| 112 | 54415 | 48 | 0,80 |
| 114 | 51356 | 50 | 0,83 |
| 116 | 48785 | 52 | 0,87 |
| 118 | 46623 | 54 | 0,90 |
| 120 | 44591 | 56 | 0,93 |
| 122 | 42205 | 58 | 0,97 |
| 124 | 40184 | 60 | 1,00 |
| 126 | 38691 | 62 | 1,03 |
| 128 | 36995 | 64 | 1,07 |
| 130 | 34916 | 66 | 1,10 |
| 132 | 32888 | 68 | 1,13 |
| 134 | 31339 | 70 | 1,17 |
| 136 | 29436 | 72 | 1,20 |
| 138 | 27786 | 74 | 1,23 |
| 140 | 26446 | 76 | 1,27 |
| 142 | 25425 | 78 | 1,30 |
| 144 | 24274 | 80 | 1,33 |
| 146 | 23713 | 82 | 1,37 |
| 148 | 22487 | 84 | 1,40 |
| 150 | 21028 | 86 | 1,43 |
| 152 | 19540 | 88 | 1,47 |
| 154 | 18531 | 90 | 1,50 |
| 156 | 18330 | 92 | 1,53 |
| 158 | 17988 | 94 | 1,57 |
| 160 | 17124 | 96 | 1,60 |
| 162 | 15875 | 98 | 1,63 |
| 164 | 14944 | 100 | 1,67 |
| 166 | 14092 | 102 | 1,70 |
| 168 | 13539 | 104 | 1,73 |
| 170 | 13090 | 106 | 1,77 |
| 172 | 12944 | 108 | 1,80 |
| 174 | 12269 | 110 | 1,83 |
| 176 | 11595 | 112 | 1,87 |
| 178 | 11391 | 114 | 1,90 |
| 180 | 10898 | 116 | 1,93 |
| 182 | 10439 | 118 | 1,97 |
| 184 | 10014 | 120 | 2,00 |
| 186 | 9555 | 122 | 2,03 |
| 188 | 9227 | 124 | 2,07 |
| 190 | 8915 | 126 | 2,10 |
| 192 | 8341 | 128 | 2,13 |
| 194 | 7925 | 130 | 2,17 |
| 196 | 7646 | 132 | 2,20 |
| 198 | 7304 | 134 | 2,23 |
| 200 | 6949 | 136 | 2,27 |
| 202 | 6574 | 138 | 2,30 |
| 204 | 6294 | 140 | 2,33 |
| 206 | 5852 | 142 | 2,37 |
| 208 | 5755 | 144 | 2,40 |
| 210 | 5505 | 146 | 2,43 |
| 212 | 5221 | 148 | 2,47 |
| 214 | 5155 | 150 | 2,50 |
| 216 | 4828 | 152 | 2,53 |
| 218 | 4668 | 154 | 2,57 |
| 220 | 4511 | 156 | 2,60 |
| 222 | 4427 | 158 | 2,63 |
| 224 | 4277 | 160 | 2,67 |
| 226 | 4161 | 162 | 2,70 |
| 228 | 4032 | 164 | 2,73 |
| 230 | 3712 | 166 | 2,77 |
| 232 | 3400 | 168 | 2,80 |
| 234 | 3261 | 170 | 2,83 |
| 236 | 3139 | 172 | 2,87 |
| 238 | 3010 | 174 | 2,90 |
| 240 | 2905 | 176 | 2,93 |
| 242 | 2809 | 178 | 2,97 |
| 244 | 2727 | 180 | 3,00 |
| 246 | 2577 | 182 | 3,03 |
| 248 | 2449 | 184 | 3,07 |
| 250 | 2391 | 186 | 3,10 |
| 252 | 2252 | 188 | 3,13 |
| 254 | 2098 | 190 | 3,17 |
| 256 | 2053 | 192 | 3,20 |
| 258 | 1983 | 194 | 3,23 |
| 260 | 1882 | 196 | 3,27 |
| 262 | 1843 | 198 | 3,30 |
| 264 | 1725 | 200 | 3,33 |
| 266 | 1558 | 202 | 3,37 |
| 268 | 1521 | 204 | 3,40 |
| 270 | 1453 | 206 | 3,43 |
| 272 | 1360 | 208 | 3,47 |
| 274 | 1317 | 210 | 3,50 |
| 276 | 1259 | 212 | 3,53 |
| 278 | 1181 | 214 | 3,57 |
| 280 | 1130 | 216 | 3,60 |
| 282 | 1088 | 218 | 3,63 |
| 284 | 1043 | 220 | 3,67 |
| 286 | 982 | 222 | 3,70 |
| 288 | 939 | 224 | 3,73 |
| 290 | 891 | 226 | 3,77 |
| 292 | 851 | 228 | 3,80 |
| 294 | 819 | 230 | 3,83 |
| 296 | 774 | 232 | 3,87 |
| 298 | 698 | 234 | 3,90 |
| 300 | 663 | 236 | 3,93 |
| 302 | 640 | 238 | 3,97 |
| 304 | 610 | 240 | 4,00 |
| 306 | 576 | 242 | 4,03 |
| 308 | 556 | 244 | 4,07 |
| 310 | 538 | 246 | 4,10 |
| 312 | 521 | 248 | 4,13 |
| 314 | 492 | 250 | 4,17 |
| 316 | 456 | 252 | 4,20 |
| 318 | 434 | 254 | 4,23 |
| 320 | 408 | 256 | 4,27 |
| 322 | 377 | 258 | 4,30 |
| 324 | 350 | 260 | 4,33 |
| 326 | 329 | 262 | 4,37 |
| 328 | 304 | 264 | 4,40 |
| 330 | 291 | 266 | 4,43 |
| 332 | 274 | 268 | 4,47 |
| 334 | 251 | 270 | 4,50 |
| 336 | 246 | 272 | 4,53 |
| 338 | 230 | 274 | 4,57 |
| 340 | 200 | 276 | 4,60 |
| 342 | 185 | 278 | 4,63 |
| 344 | 168 | 280 | 4,67 |
| 346 | 149 | 282 | 4,70 |
| 348 | 139 | 284 | 4,73 |
| 350 | 123 | 286 | 4,77 |
| 352 | 112 | 288 | 4,80 |
| 354 | 106 | 290 | 4,83 |
| 356 | 92 | 292 | 4,87 |
| 358 | 83 | 294 | 4,90 |
| 360 | 71 | 296 | 4,93 |
| 362 | 57 | 298 | 4,97 |
| 364 | 47 | 300 | 5,00 |
| 366 | 39 | 302 | 5,03 |
| 368 | 33 | 304 | 5,07 |
| 370 | 27 | 306 | 5,10 |
| 372 | 21 | 308 | 5,13 |
| 374 | 15 | 310 | 5,17 |
| 376 | 11 | 312 | 5,20 |
| 378 | 8 | 314 | 5,23 |
| 380 | 5 | 316 | 5,27 |
| 382 | 4 | 318 | 5,30 |
| 384 | 3 | 320 | 5,33 |
| 386 | 4 | 322 | 5,37 |
| 388 | 3 | 324 | 5,40 |
| 390 | 3 | 326 | 5,43 |
| 392 | 3 | 328 | 5,47 |
| 394 | 3 | 330 | 5,50 |
| 396 | 3 | 332 | 5,53 |
| 398 | 3 | 334 | 5,57 |
| 400 | 3 | 336 | 5,60 |
| 402 | 3 | 338 | 5,63 |
| 404 | 3 | 340 | 5,67 |
| 406 | 2 | 342 | 5,70 |
| 408 | 2 | 344 | 5,73 |
| 410 | 1 | 346 | 5,77 |
| 412 | 2 | 348 | 5,80 |
| 414 | 1 | 350 | 5,83 |

### Beschreibung der Figuren

**Fig.1**
   Schematische Übersicht der Anordnung der Stromquellen für den "Power"-Brennprozess des erfindungsgemäßen Trockenaerosol-Generators
   Fig 1A: Versuchsvariante 1
   Fig 1B: Versuchsvariante 2A und 2B
   Fig 1C: Versuchsvariante 3A und 3B
   Fig.1D: Versuchsvariante 4
**Fig.2**
   Vergleichsmessungen zur Darstellung Wirksamkeit der entwickelten "intelligenten Powerstrom-Elektronik (IPE)" im Trockenaerosolgenerator
**Fig.3**
   Monitoring der Radioaktivitätswerte bei der Trockenaerosol Zubereitung im Generator
**Fig.4**
   Ergebnisse der Messung für die Überwachung des Restsauerstoffsgehaltes beim Aufbau der Inertgas-Schutzgasatmosphäre der Brennkammer des Trockenaerosol-Generators (Auflösung des Diagramms "y in 10.000")
**Fig.5**
   Ergebnisse der Messung für die Überwachung des Restsauerstoffsgehaltes beim Aufbau der Inertgas-Schutzgasatmosphäre der Brennkammer des TrockenaerosolGenerator (Auflösung des Diagramms "y in 100") Detailansicht

### Bezugszeichen

- **1**: Stromquelle(n)
- **2**: Stromaufbereitungseinheit
- **3**: Stromquellen-Kombinationseinheit
- **4**: Intelligente Powerstrom-Elektronik
- **5**: Power-Transformator
- **6**: Generator-Brennkammer

## Patentansprüche

1. Vorrichtung zur Erzeugung eines radioaktiv-markierten Trockenaerosols umfassend
- einen Kohlenstofftiegel mit einem offenen Hohlraum geeignet zur Befüllung mit radioaktivem Nuklid,
- eine Brennkammer, welche geeignet ist, einen Kohlenstofftiegel aufzunehmen,
- einen Impulsstrom-Geber, welcher eine blitzartige Erhitzung des Kohlenstofftiegels und eines darin enthaltenen radioaktiven Nuklids ermöglicht, sodass ein Trockenaerosol aus radioaktiv-markierten Kohlenstoffpartikeln gebildet wird, welches anschließend zur medizinischen Bildgebung aus der Vorrichtung in die Lunge eines Patienten geleitet werden kann,
- eine Steuerungseinheit,
wobei das Füllvolumen des Kohlenstofftiegels 0,1 mL - 1 mL, vorzugsweise mindestens 0,2 mL, mindestens 0,4 mL, mindestens 0,5 mL oder mindestens 0,6 mL, besonders bevorzugt 0,3 - 0,6 mL und noch bevorzugter 0,4 - 0,5 mL beträgt, und **gekennzeichnet dadurch, dass** die Vorrichtung folgendes umfasst:
- eine Sonde zur Messung des Sauerstoffgehaltes in der Brennkammer,
- eine Vakuumpumpe zur Entlüftung der Brennkammer,
- einen Druckluftkompressor zur Belüftung der Brennkammer,
- eine Kamera, welche eine Überwachung der Befüllung des Kohlenstofftiegels ermöglicht,
- eine Gamma-Zähler-Messeinheit, welche geeignet ist, eine Veränderung der Radioaktivitätsmenge im Kohlenstofftiegel und/oder der Brennkammer zu messen,
- ein Flow-Meter, welches konfiguriert ist, das Atemvolumen eines Patienten zu bestimmen,
- mindestens eine Batterie und/oder PowerCap/Pufferkondensator und/oder Hochleistungskondensator,
- einen Wechselrichter zur Transformation des Gleichstroms einer Batterie und/oder eines PowerCap/Pufferkondensator und/oder eines Hochleistungskondensators in eine Wechselspannungs-Sinuswelle,
- eine intelligente Netzelektronik-Einheit für einen Stromnetzbetrieb der Vorrichtung mit Trafo, welche konfiguriert ist, die Sinuswelle der Wechselspannung des Netzstroms zu analysieren, wobei die Sinuswelle vorzugsweise eine Standardfrequenz von 50 - 60 Hz aufweist und die Wechselspannung des Netzstroms vorzugsweise 100 bis 400 Volt, vorzugsweise 110 - 260 Volt, insbesondere 230 Volt beträgt,
- einen externen mit der Vorrichtung verbundener Radioaktivitäts-Sensor, welcher konfiguriert ist, die Radioaktivitätszunahme in der Lunge des Patienten bei einer Verabreichung des erzeugten radioaktiven Trockenaerosols zu erfassen,
- optional eine Vorrichtung zur Integration einer Argon- oder Schutzgas-Quelle,
- optional einen externen Beatmungsbeutel, welcher an die Vorrichtung angeschlossen werden kann und konfiguriert ist, das Volumen des von einem Patienten eingeatmeten radioaktiv-markierten Trockenaerosols zu vergrößern.

2. Vorrichtung gemäß Anspruch 1, wobei das radioaktive Nuklid ausgewählt ist aus Technetium (Tc-99m), Gallium 68, Molybdän-99, Chrom-51, Holmium-166, Rubidium-82, Eisen-59, Lutetium-177, Palladium-103, Kalium-42, Scandium-47, Selen-75, Natrium-24, Xenon-133, Ytterbium-169, Ytterbium-177, Jod-131, Jod-125, Samarium-153, Rhenium-186, Lutetium-177, Phosphor-32, Cäsium-131, wobei Technetium (Tc-99m) und Gallium 68 besonders bevorzugt sind.

3. Vorrichtung gemäß Anspruch 1 oder 2, wobei das Gerät vorzugsweise keine Wechselkontakte umfasst, die vom Anwender getauscht werden müssen.

4. Vorrichtung gemäß einem der vorherigen Ansprüche, wobei das radioaktiv-markierte Trockenaerosol Nanopartikel umfasst, wobei die Nanopartikel vorzugsweise einen durchschnittlichen Durchmesser zwischen 5 - 250 nm, insbesondere 10 - 100 nm, besonders bevorzugt 30 - 60 nm aufweisen.

5. Vorrichtung gemäß dem vorherigen Anspruch, wobei mindestens 75% der Nanopartikel einen Durchmesser kleiner als 100 nm aufweisen.

6. Vorrichtung gemäß einem der vorherigen Ansprüche, wobei die Vorrichtung Mittel umfasst, um den Kohlenstofftiegel automatisch in Kontakt mit elektrischen Kontakten zu bringen, wobei die Mittel vorzugsweise auch für ein automatisches "Einschleifen" der Kontaktierung zwischen Tiegel und den elektrischen Kontakten konfiguriert sind.

7. Verfahren zur Erzeugung von radioaktiv-markiertem Trockenaerosol umfassend
a. Bereitstellen einer Vorrichtung zur Erzeugung von radioaktiv-markiertem Trockenaerosol gemäß Anspruch 1,
b. Durchführung eines Atemtests (Pre-Breathing) des Pateienten, wobei das Atemvolumen des Patienten zur Beurteilung seiner Untersuchungsfähigkeit gemessen wird,
c. Kamera-überwachtes Befüllen eines in die Brennkammer der Vorrichtung eingesetzten Kohlenstofftiegels mit einem radioaktiven Nuklid,
d. optionaler Drucktest zur Kontrolle der Dichtigkeit der Brennkammer,
e. optionales Verdampfen einer Trägerflüssigkeit des radioaktiven Nuklids,
f. optionales Trocknen des radioaktiven Nuklids,
g. Evakuieren der Luft aus der Brennkammer zur Erzeugung eines Vakuums,
h. Befüllen der Brennkammer mit Argongas, mit gleichzeitiger Überwachung von eventuell vorhandenem Restsauerstoff, zur Erzeugung einer inerten Argon-, Stickstoff- oder Schutzgas-Atmosphäre in der Brennkammer,
i. Vorwärmen des Kohlenstofftiegels auf vorzugsweise 500°C,
j. Erhitzung des befüllten Kohlenstofftiegels auf vorzugsweise 2.750 °C zur Erzeugung eines radioaktiv-markierten Trockenaerosols oder Nanopartikel-Komposits, wobei der während des Aufheizens benötigte Strom zur Verfügung gestellt wird, entweder
I. durch die enthaltene mindestens eine Batterie und/oder PowerCap/Pufferkondensator und/oder Hochleistungskondensator, oder
II. durch Netzstrom, welcher durch die mindestens eine Batterie und/oder PowerCap/Pufferkondensator und/oder Hochleistungskondensator unterstützt und optional verstärkt wird, oder
III. durch Netzstrom, wobei die intelligente Netzelektronik-Einheit die Sinuswelle (vorzugsweise 50 - 60 Hz) der Wechselspannung des Netzstroms (vorzugsweise 100 - 400 Volt, bevorzugter 110 - 260 Volt, insbesondere 230 Volt) analysiert, und den Trafo erst einschaltet, wenn die Einschaltlast des Trafos einen vordefinierten, günstigen Zeitpunkt auf der Sinuswelle des Netzstroms erreicht,
sodass eine Erhitzung des befüllten Kohlenstofftiegels auf 2.500 - 3.000 °C, insbesondere 2.750 °C vorzugsweise in weniger als 2 Sekunden, vorzugsweise bis zu 1,8 Sekunden, vorzugsweise bis zu 1,5 Sekunden, vorzugsweise bis zu 1,2 Sekunden, vorzugsweise bis zu 1 Sekunde, vorzugsweise bis zu 0,8 Sekunden, noch bevorzugter bis zu 0,6 Sekunden und besonders bevorzugt bis zu 0,5 Sekunden erreicht wird,
k. optionale Überwachung der Veränderung der Radioaktivität in der Brennkammer,
l. Lungen-Aktivitäts-Überwachung während der Inhalation des erzeugten radioaktiv-markierten Trockenaerosols oder Nanopartikel-Komposits durch einen Patienten, wobei die vom Patienten inhalierte Radioaktivitätsmenge durch einen, vorzugsweise am Patienten lokalisierten, Radioaktivitäts-Sensor überwacht wird,
m. Spülung der Brennkammer mit Druckluft zur Entfernung der radioaktiven Partikel, wobei die radioaktiven Partikel von einem Filter aus dem ausströmenden Luft-, Argon-, Stickstoff- oder Schutzgasgemisch entfernt werden, und optionale Überwachung der Veränderung der Radioaktivität in der Brennkammer,
n. optionale automatische Protokollierung einer oder mehrerer Vorgangsparameter.

8. Verfahren gemäß dem vorherigen Anspruch wobei das radioaktive Nuklid ausgewählt ist aus Technetium (Tc-99m), Gallium 68, Molybdän-99, Chrom-51, Holmium-166, Rubidium-82, Eisen-59, Lutetium-177, Palladium-103, Kalium-42, Scandium-47, Selen-75, Natrium-24, Xenon-133, Ytterbium-169, Ytterbium-177, Jod-131, Jod-125, Samarium-153, Rhenium-186, Lutetium-177, Phosphor-32, Cäsium-131, wobei Technetium (Tc-99m) und Gallium 68 besonders bevorzugt sind.

9. Verfahren gemäß einem der Ansprüche 7 - 8, wobei während der Erhitzung des befüllten Kohlenstofftiegels unter Punkt j. der Gleichstrom der mindestens einen Batterie und/oder PowerCap/Pufferkondensator und/oder Hochleistungskondensators in eine Wechselspannung (mit vorzugsweise 230 Volt) mittels Pulsweitenmodulation oder Wechselrichter umgewandelt wird, sodass die aus der mindestens einen Batterie und/oder PowerCap/Pufferkondensator und/oder Hochleistungskondensator gewandelte Spannung auf die Sinuswelle der Wechselspannung (vorzugsweise 230 Volt) des Stromnetzeingangs der Vorrichtung aufgeschaltet wird und somit die Netzstromstärke auf vorzugsweise ≥ 16 Amper verstärkt.

10. Verfahren gemäß einem der Ansprüche 7 - 9, wobei während des Atemtests unter Punkt b. der Patient durch ein, vorzugsweise an die Vorrichtung angeschlossenes, Schlauchset atmet, sodass beim Einatmen gefilterte Raumluft durch ein, vorzugsweise in der Vorrichtung integriertes, Flow-Meter strömt, welches das eingeatmete Volumen pro Atemzug misst.

## Claims

1. Apparatus for producing a radiolabeled dry aerosol comprising
- a carbon crucible with an open cavity suitable for filling with radioactive nuclide,
- a firing chamber which is suitable for receiving a carbon crucible,
- a pulse current generator which enables an instantaneous heating of the carbon crucible and a radioactive nuclide contained therein, such that a dry aerosol of radiolabeled carbon particles is formed, which can subsequently be passed from the apparatus into the lung of a patient for medical imaging,
- a control unit,
- wherein the filling volume of the carbon crucible is 0.1 mL - 1 mL, preferably at least 0.2 mL, at least 0.4 mL, at least 0.5 mL or at least 0.6 mL, more preferably 0.3 - 0.6 mL and even more preferably 0.4 - 0.5 mL, and
**characterized in that**
the apparatus comprises the following:
- a probe for measuring the oxygen content in the firing chamber,
- a vacuum pump for evacuating the firing chamber,
- a compressed-air compressor for ventilating the firing chamber,
- a camera which enables a monitoring the filling of the carbon crucible,
- a gamma counter measuring unit which is suitable for measuring a change in the amount of radioactivity in the carbon crucible and/or the firing chamber,
- a flow meter that is configured to determine a patient's respiratory volume,
- at least one battery and/or PowerCap/buffer capacitor and/or high-performance capacitor,
- an inverter for transforming the direct current of a battery and/or PowerCap/buffer capacitor and/or high-performance capacitor into an alternating voltage sine wave,
- an intelligent mains electronics unit for mains operation of the apparatus with transformer, which is configured to analyze the sine wave of the alternating voltage of the mains current, wherein the sine wave preferably has a standard frequency of 50 - 60 Hz and the alternating voltage of the mains current is preferably 100 to 400 volts, preferably 110 - 260 volts, in particular 230 volts,
- an external radioactivity sensor connected to the apparatus which is configured to detect the increase in radioactivity in the lung of the patient upon administration of the produced radioactive dry aerosol,
- optionally an apparatus for integrating an argon or protecting gas source,
- optionally an external ventilation bag which can be connected to the apparatus and is configured to increase the volume of radiolabeled dry aerosol inhaled by a patient.

2. Apparatus according to claim 1, wherein the radioactive nuclide is selected from technetium (Tc-99m), gallium-68, molybdenum-99, chromium-51, holmium-166, rubidium-82, iron-59, lutetium-177, palladium-103, potassium-42, scandium-47, selenium-75, sodium-24, xenon-133, ytterbium-169, ytterbium-177, iodine-131, iodine-125, samarium-153, rhenium-186, lutetium-177, phosphorus-32, caesium-131, wherein technetium (Tc-99m) and gallium 68 are particularly preferred.

3. Apparatus according to claim 1 or 2, wherein the apparatus preferably does not comprise interchangeable contacts which have to be replaced by the user.

4. Apparatus according to any one of the preceding claims, wherein the radiolabeled dry aerosol comprises nanoparticles, wherein the nanoparticles preferably have an average diameter between 5 - 250 nm, in particular 10 - 100 nm, more preferably 30 - 60 nm.

5. Apparatus according to the previous claim, wherein at least 75% of the nanoparticles have a diameter of less than 100 nm.

6. Apparatus according to any one of the preceding claims, wherein the apparatus comprises means for automatically bringing the carbon crucible into contact with electrical contacts, wherein the means are preferably also configured for automatic "grinding-in" of the contact between the crucible and the electrical contacts.

7. Method for producing radiolabeled dry aerosol comprising
a. providing an apparatus for producing radiolabeled dry aerosol according to claim 1,
b. carrying out a breathing test (pre-breathing) on the patient, wherein the respiratory volume of the patient is measured in order to assess their fitness for examination,
c. camera-monitored filling of a carbon crucible inserted into the firing chamber of the apparatus with a radioactive nuclide,
d. optional pressure test for checking the leak-tightness of the firing chamber,
e. optional evaporation of a carrier liquid of the radioactive nuclide,
f. optional drying of the radioactive nuclide,
g. evacuation of the air from the firing chamber to generate a vacuum,
h. filling the firing chamber with argon gas, with simultaneous monitoring of eventually present residual oxygen, to generate an inert argon, nitrogen or protecting gas atmosphere in the firing chamber,
i. preheating the carbon crucible to preferably 500°C,
j. heating the filled carbon crucible to preferably 2,750 °C to generate a radiolabeled dry aerosol or nanoparticle composite, wherein the current required during heating is provided, either
I. by the at least one battery and/or PowerCap/buffer capacitor and/or high-performance capacitor contained therein, or
II. by mains current, which is supported and optionally amplified by at least one battery and/or PowerCap/buffer capacitor and/or high-performance capacitor, or
III. by mains current, wherein the intelligent mains electronics unit analyzes the sine wave (preferably 50 - 60 Hz) of the alternating voltage of the mains current (preferably 100 - 400 volts, more preferably 110 - 260 volts, in particular 230 volts) and switches on the transformer only when the switch-on load of the transformer reaches a predefined, favorable point in time on the sine wave of the mains current,
such that a heating of the filled carbon crucible to 2,500 - 3,000 °C, in particular 2,750 °C, is achieved preferably in less than 2 seconds, preferably up to 1.8 seconds, preferably up to 1.5 seconds, preferably up to 1.2 seconds, preferably up to 1 second, preferably up to 0.8 seconds, even more preferably up to 0.6 seconds and particularly preferably up to 0.5 seconds,
k. optional monitoring of the change in radioactivity in the firing chamber,
l. lung activity monitoring during inhalation of the generated radiolabeled dry aerosol or nanoparticle composite by a patient, wherein the amount of radioactivity inhaled by the patient is monitored by a radioactivity sensor, preferably localized on the patient,
m. purging of the firing chamber with compressed air to remove the radioactive particles, wherein the radioactive particles are removed by a filter from the outflowing air, argon, nitrogen or protection gas mixture, and optionally monitoring of the change in radioactivity in the firing chamber,
n. optional automatic logging of one or more process parameters.

8. Method according to the previous claim wherein the radioactive nuclide is selected from technetium (Tc-99m), gallium-68, molybdenum-99, chromium-51, holmium-166, rubidium-82, iron-59, lutetium-177, palladium-103, potassium-42, scandium-47, selenium-75, sodium-24, xenon-133, ytterbium-169, ytterbium-177, iodine-131, iodine-125, samarium-153, rhenium-186, lutetium-177, phosphorus-32, caesium-131, wherein technetium (Tc-99m) and gallium 68 are particularly preferred.

9. Method according to any one of claims 7 - 8, wherein during the heating of the filled carbon crucible under item j. the direct current of the at least one battery and/or PowerCap/buffer capacitor and/or high-performance capacitor is converted into an alternating voltage (preferably 230 volts) by means of pulse width modulation or an inverter, such that the voltage converted from the at least one battery and/or PowerCap/buffer capacitor and/or high-performance capacitor is applied to the sine wave of the alternating voltage (preferably 230 volts) of the mains current input of the apparatus and thus amplifies the mains current to preferably ≥ 16 amperes.

10. Method according to one of claims 7 - 9, wherein during the breathing test under item b. the patient breathes through a tube set, preferably connected to the apparatus, such that when breathing in, filtered room air flows through a flow meter, preferably integrated in the apparatus, which measures the inhaled volume per breath.

## Revendications

1. Dispositif pour la génération d'un aérosol sec marqué radioactivement, comprenant
- un creuset en carbone comportant une cavité ouverte, apte à être remplie d'un nucléide radioactif ,
- une chambre de combustion, apte à recevoir un creuset en carbone ,
- un générateur de courant impulsionnel, permettant un échauffement de type éclair du creuset en carbone et d'un nucléide radioactif qu'il contient, de sorte qu'il se forme un aérosol sec de particules de carbone marquées radioactivement, lequel peut ensuite être acheminé, à partir du dispositif, dans les poumons d'un patient en vue d'une imagerie médicale ,
- une unité de commande,
dans lequel
le volume de remplissage du creuset en carbone est de 0,1 mL à 1 mL, de préférence d'au moins 0,2 mL, d'au moins 0,4 mL, d'au moins 0,5 mL ou d'au moins 0,6 mL, de manière particulièrement préférée de 0,3 mL à 0,6 mL et de manière davantage préférée encore de 0,4 mL à 0,5 mL, et **caractérisé en ce que** le dispositif comprend :
- une sonde destinée à mesurer la teneur en oxygène dans la chambre de combustion ,
- une pompe à vide destinée à évacuer l'air de la chambre de combustion ,
- un compresseur d'air comprimé destiné à ventiler la chambre de combustion ,
- une caméra permettant de surveiller le remplissage du creuset en carbone ,
- une unité de mesure à compteur gamma, apte à mesurer une variation de la quantité de radioactivité dans le creuset en carbone et/ou dans la chambre de combustion ,
- un débitmètre configuré pour déterminer le volume respiratoire d'un patient,
- au moins une batterie et/ou un PowerCap / condensateur tampon et/ou un condensateur de forte puissance,
- un onduleur destiné à transformer le courant continu provenant d'une batterie et/ou d'un PowerCap / condensateur tampon et/ou d'un condensateur de forte puissance en une onde sinusoïdale de tension alternative,
- une unité intelligente d'électronique d'alimentation pour le fonctionnement du dispositif sur le réseau électrique, comportant un transformateur, configurée pour analyser l'onde sinusoïdale de la tension alternative du courant du réseau, dans lequel l'onde sinusoïdale présente de préférence une fréquence normalisée de 50 à 60 Hz, et la tension alternative du courant de réseau est de préférence comprise entre 100 et 400 V, de préférence entre 110 et 260 V, en particulier de 230 V,
- un capteur de radioactivité externe relié au dispositif, configuré pour détecter l'augmentation de la radioactivité dans les poumons du patient lors de l'administration de l'aérosol sec radioactif généré,
- éventuellement un dispositif destiné à intégrer une source d'argon ou de gaz de protection,
- éventuellement un ballon de ventilation externe, pouvant être raccordé au dispositif et configuré pour augmenter le volume de l'aérosol sec marqué radioactivement inhalé par un patient.

2. Dispositif selon la revendication 1, dans lequel le nucléide radioactif est choisi parmi le technétium (Tc-99m), le gallium-68, le molybdène-99, le chrome-51, l'holmium-166, le rubidium-82, le fer-59, le lutétium-177, le palladium-103, le potassium-42, le scandium-47, le sélénium-75, le sodium-24, le xénon-133, l'ytterbium-169, l'ytterbium-177, l'iode-131, l'iode-125, le samarium-153, le rhénium-186, le lutétium-177 et le phosphore-32, ainsi que le césium-131, dans lequel le technétium (Tc-99m) et le gallium-68 sont particulièrement préférés.

3. Dispositif selon la revendication 1 ou 2, dans lequel le dispositif ne comprend de préférence aucun contact électrique remplaçable devant être remplacé par l'utilisateur.

4. Dispositif selon l'une quelconque des revendications précédentes, dans lequel l'aérosol sec marqué radioactivement comprend des nanoparticules, dans lequel les nanoparticules présentent de préférence un diamètre moyen compris entre 5 et 250 nm, en particulier entre 10 et 100 nm, de préférence tout particulièrement entre 30 et 60 nm.

5. Dispositif selon la revendication précédente, dans lequel au moins 75 % des nanoparticules présentent un diamètre inférieur à 100 nm.

6. Dispositif selon l'une quelconque des revendications précédentes, dans lequel le dispositif comprend des moyens permettant de mettre automatiquement le creuset en carbone en contact avec des contacts électriques, dans lequel les moyens sont de préférence également configurés pour assurer un « rodage » automatique du contact électrique entre le creuset et les contacts électriques.

7. Procédé de génération d'un aérosol sec marqué radioactivement, comprenant
a. la mise à disposition d'un dispositif de génération d'un aérosol sec marqué radioactivement selon la revendication 1 ,
b. la réalisation d'un test respiratoire du patient, dans lequel le volume respiratoire du patient est mesuré afin d'évaluer son aptitude à subir l'examen,
c. le remplissage, sous surveillance par caméra, d'un creuset en carbone disposé dans la chambre de combustion du dispositif avec un nucléide radioactif,
d. un test de pression optionnel destiné à contrôler l'étanchéité de la chambre de combustion,
e. l'évaporation optionnelle d'un liquide vecteur du nucléide radioactif,
f. le séchage optionnel du nucléide radioactif,
g. l'évacuation de l'air de la chambre de combustion afin de créer un vide,
h. le remplissage de la chambre de combustion avec de l'argon, avec surveillance simultanée de la présence éventuelle d'oxygène résiduel, afin de créer une atmosphère inerte d'argon, d'azote ou de gaz de protection dans la chambre de combustion,
i. le préchauffage du creuset en carbone à une température de préférence de 500 °C,
j. le chauffage du creuset en carbone rempli de préférence à 2 750 °C pour produire un aérosol sec ou un composite de nanoparticules marqué radioactivement, dans lequel le courant nécessaire au chauffage est fourni
I. soit par l'au moins une batterie et/ou un PowerCap / condensateur tampon et/ou un condensateur de forte puissance ; ou
II. soit par le courant du réseau, lequel est assisté et, de manière optionnelle, renforcé par l'au moins une batterie et/ou un PowerCap / condensateur tampon et/ou un condensateur de forte puissance ; ou
III. soit par le courant du réseau, dans lequel l'unité intelligente d'électronique d'alimentation analyse l'onde sinusoïdale (de préférence de 50 à 60 Hz) de la tension alternative du courant du réseau (de préférence de 100 à 400 V, de manière davantage préférée de 110 à 260 V, en particulier de 230 V), et ne met le transformateur sous tension que lorsque la charge d'enclenchement du transformateur atteint un instant prédéfini favorable sur l'onde sinusoïdale du courant du réseau, de sorte que le creuset en carbone rempli soit chauffé à 2 500 - 3 000 °C, en particulier à 2 750 °C, de préférence en moins de 2 secondes, de préférence jusqu'à 1,8 seconde, de préférence jusqu'à 1,5 seconde, de préférence jusqu'à 1,2 seconde, de préférence jusqu'à 1 seconde, de préférence jusqu'à 0,8 seconde, plus particulièrement jusqu'à 0,6 seconde et surtout jusqu'à 0,5 seconde,
k. la surveillance, de manière optionnelle, de la variation de la radioactivité dans la chambre de combustion,
l. la surveillance de l'activité pulmonaire pendant l'inhalation, par un patient, de l'aérosol sec marqué radioactivement ou du composite de nanoparticules généré, dans lequel la quantité de radioactivité inhalée par le patient est surveillée au moyen d'un capteur de radioactivité, de préférence disposé sur le patient,
m. le rinçage de la chambre de combustion à l'air comprimé afin d'éliminer les particules radioactives, dans lequel les particules radioactives sont retirées du mélange gazeux sortant d'air, d'argon, d'azote ou de gaz de protection par un filtre, et la surveillance, de manière optionnelle, de la variation de la radioactivité dans la chambre de combustion,
n. la consignation automatique, de manière optionnelle, d'un ou de plusieurs paramètres du procédé.

8. Procédé selon la revendication précédente, dans lequel le nucléide radioactif est choisi parmi le technétium (Tc-99m), le gallium-68, le molybdène-99, le chrome-51, l'holmium-166, le rubidium-82, le fer-59, le lutétium-177, le palladium-103, le potassium-42, le scandium-47, le sélénium-75, le sodium-24, le xénon-133, l'ytterbium-169, l'ytterbium-177, l'iode-131, l'iode-125, le samarium-153, le rhénium-186, le lutétium-177 et le phosphore-32, ainsi que le césium-131, dans lequel le technétium (Tc-99m) et le gallium-68 sont particulièrement préférés.

9. Procédé selon l'une quelconque des revendications 7 à 8, dans lequel, pendant l'échauffement du creuset en carbone rempli selon le point j., le courant continu de l'au moins une batterie et/ou un PowerCap / condensateur tampon et/ou un condensateur de forte puissance est converti en une tension alternative (de préférence de 230 V) au moyen d'une modulation de largeur d'impulsion ou d'un onduleur, de sorte que la tension convertie provenant de l'au moins une batterie et/ou un PowerCap / condensateur tampon et/ou un condensateur de forte puissance est superposée à l'onde sinusoïdale de la tension alternative (de préférence de 230 V) de l'entrée secteur du dispositif, augmentant ainsi l'intensité du courant du réseau, de préférence jusqu'à ≥ 16 A.

10. Procédé selon l'une quelconque des revendications 7 à 9, dans lequel, pendant le test respiratoire de l'étape b., le patient respire au moyen d'un ensemble de tuyaux, de préférence raccordé au dispositif, de sorte que, lors de l'inspiration, de l'air ambiant filtré s'écoule à travers un débitmètre, de préférence intégré au dispositif, lequel mesure le volume inspiré à chaque respiration.
